(19) Europäisches Patentamt
European Patent Office
Office européen des brevets

(11) Veröffentlichungsnummer: **0 207 285**
**A1**

(12) # EUROPÄISCHE PATENTANMELDUNG

(21) Anmeldenummer: 86107125.6

(22) Anmeldetag: 26.05.86

(51) Int. Cl.4: C 07 D 401/04
A 01 N 43/56

(30) Priorität: 07.06.85 DE 3520330

(43) Veröffentlichungstag der Anmeldung:
07.01.87 Patentblatt 87/2

(84) Benannte Vertragsstaaten:
AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Schallner, Otto, Dr.
Noldeweg 22
D-4019 Monheim(DE)

(72) Erfinder: Gehring, Reinhold, Dr.
Dasnöckel 49
D-5600 Wuppertal 11(DE)

(72) Erfinder: Stetter, Jörg, Dr.
Gellertweg 4
D-5600 Wuppertal 1(DE)

(72) Erfinder: Santel, Hans-Joachim, Dr.
Grünstrasse 9a
D-5090 Leverkusen 1(DE)

(72) Erfinder: Schmidt, Robert R., Dr.
Im Waldwinkel 110
D-5060 Bergisch Gladbach 2(DE)

(54) **5-Amino-1-pyridyl-pyrazole.**

(57) Die Erfindung betrifft neue 5-Amino-1-pyridyl-pyrazole der Formel (I),

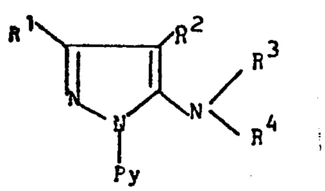

(I)

in welcher

$R^1$ für Wasserstoff oder für Alkyl mit 1 bis 12 Kohlenstoffatomen steht,

$R^2$ für Wasserstoff, Nitro, Nitroso, Halogen oder für einen Rest

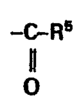

steht, wobei

$R^5$ für Wasserstoff, Hydroxy, Alkyl, Alkenyl, Alkinyl, Halogenalkyl, Alkoxyalkyl, Alkylthioalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht,

$R^3$ für Wasserstoff, für einen Rest

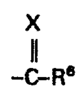

oder für einen Rest $-S(O)_n-R^7$, steht

$R^4$ für Wasserstoff, für Alkyl, für einen Rest

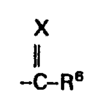

oder für einen Rest $-S(O)_n-R^7$, steht, und für den Fall, daß $R^3$ für einen $-SO_2-R^7$-Rest steht auch für ein salzartig gebundenes anorganisches oder organisches Kation steht,

$R^6$ für Wasserstoff, Alkyl, Alkenyl, Alkinyl, Halogenalkyl, Alkoxyalkyl, Alkylthioalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, Dialkylami-

./...

EP 0 207 285 A1

no oder für gegebenenfalls substituiertes Arylamino steht,

X für Sauerstoff oder Schwefel steht,

n für eine Zahl 0, 1 oder 2 steht,

$R^7$ für Alkyl, Halogenalkyl oder für gegebenenfalls substituiertes Aryl steht und

Py für substituiertes C-verknüpftes Pyridyl steht, mehrere Verfähren zu ihrer Herstellung, ihre Verwendung als Herbizide und Zwischenprodukte zur Herstellung von 5-Amino-1-pyridyl-pyrazolen der Formel (I).

FIG. 1

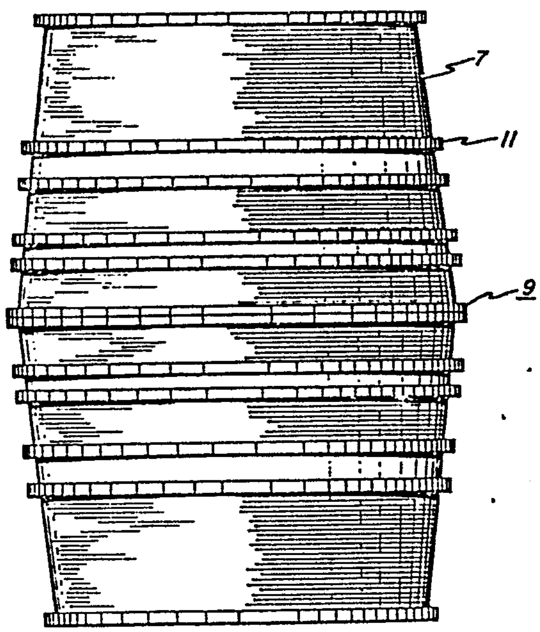

0207285

BAYER AKTIENGESELLSCHAFT 5090 Leverkusen, Bayerwerk
Konzernverwaltung RP
Patentabteilung KM/li-c
Ib

## 5-Amino-1-pyridyl-pyrazole

Die Erfindung betrifft neue 5-Amino-1-pyridyl-pyrazole, mehrere Verfahren zu ihrer Herstellung und ihre Verwendung als Herbizide.

Es ist bereits bekannt, daß bestimmte 5-Amino-1-aryl-pyrazole, wie beispielsweise das 4-Cyano-5-propion-amido-1-(2,3,4-trichlorphenyl)-pyrazol, herbizide, insbesondere auch selektiv-herbizide Eigenschaften besitzen (vergl. z.B. DE-OS 32 26 513).

Deren herbizide Wirkung gegenüber Schadpflanzen ist jedoch ebenso wie ihre Verträglichkeit gegenüber wichtigen Kulturpflanzen nicht immer in allen Anwendungsbereichenvöllig zufriedenstellend.

Es wurden neue 5-Amino-1-pyridyl-pyrazole der allgemeinen Formel (I),

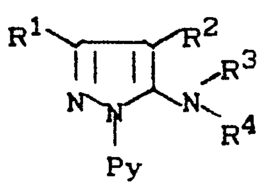

(I)

in welcher

$R^1$ für Wasserstoff oder für Alkyl mit 1 bis 12 Kohlenstoffatomen steht,

$R^2$ für Wasserstoff, Nitro, Nitroso, Halogen oder für einen Rest $-\underset{\underset{O}{\|}}{C}-R^5$ steht, wobei

$R^5$ für Wasserstoff, Hydroxy, Alkyl, Alkenyl, Alkinyl, Halogenalkyl, Alkoxyalkyl, Alkylthioalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht,

$R^3$ für Wasserstoff, für einen Rest $-\overset{\overset{X}{\|}}{C}-R^6$ oder für einen Rest $-S(O)_n-R^7$, steht,

$R^4$ für Wasserstoff, für Alkyl, für einen Rest $-\overset{\overset{X}{\|}}{C}-R^6$ oder für einen Rest $-S(O)_n-R^7$ steht, und für den Fall, daß $R^3$ für einen $-SO_2-R^7$-Rest steht auch für ein salzartig gebundenes anorganisches oder organisches Kation steht,

$R^6$ für Wasserstoff, Alkyl, Alkenyl, Alkinyl, Halogenalkyl, Alkoxyalkyl, Alkylthioalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht,

X für Sauerstoff oder Schwefel steht,

n für eine Zahl 0, 1 oder 2 steht,

$R^7$ für Alkyl, Halogenalkyl oder für gegebenenfalls substituiertes Aryl steht und

Py für substituiertes C-verknüpftes Pyridyl steht,

gefunden.

Weiterhin wurde gefunden, daß sich die neuen 5-Amino-1-pyridyl-pyrazole der allgemeinen Formel (I),

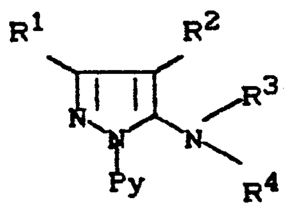

(I)

in welcher

$R^1$ für Wasserstoff oder für Alkyl mit 1 bis 12 Kohlenstoffatomen steht,

$R^2$ für Wasserstoff, Nitro, Nitroso, Halogen oder für den Rest $-\underset{\underset{O}{\|}}{C}-R^5$ steht, wobei

$R^5$ für Wasserstoff, Hydroxy, Alkyl, Alkenyl, Alkinyl, Halogenalkyl, Alkoxyalkyl, Alkylthioalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht,

$R^3$ für Wasserstoff, für einen Rest $-\overset{\overset{X}{\|}}{C}-R^6$ oder für einen Rest $-S(O)_n-R^7$ steht,

$R^4$ für Wasserstoff, für Alkyl, für einen Rest $-\overset{\overset{X}{\|}}{C}-R^6$ oder für einen Rest $-S(O)_n-R^7$ steht und für den Fall, daß $R^3$ für einen $-SO_2-R^7$-Rest steht auch für ein salzartig gebundenes anorganisches oder organisches Kation steht,

$R^6$ für Wasserstoff, Alkyl, Alkenyl, Alkinyl, Halogenalkyl, Alkoxyalkyl, Alkylthioalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht,

X für Sauerstoff oder Schwefel steht,

n für eine Zahl 0, 1 oder 2 steht,

$R^7$ für Alkyl, Halogenalkyl oder für gegebenenfalls substituiertes Aryl steht und

Py für substituiertes C-verknüpftes Pyridyl steht,

nach folgenden Verfahren herstellen lassen:

Man erhält

a) die erfindungsgemäßen 5-Amino-1-pyridyl-pyrazol-Derivate der Formel (Ia),

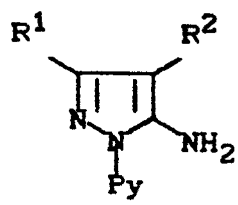

(Ia)

in welcher

$R^1$, $R^2$ und Py die oben angegebene Bedeutung haben,

wenn man Pyridylhydrazine der Formel (II),

$$Py - NH - NH_2 \qquad (II),$$

in welcher

Py die oben angegebene Bedeutung hat,

mit Acrylnitril-Derivaten der Formel (III),

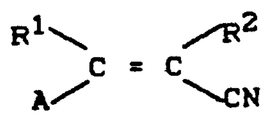

(III)

in welcher

$R^1$ und $R^2$ die oben angegebene Bedeutung haben und

A für Halogen, Hydroxy, Alkoxy oder Dialkylamino steht,

zunächst in einer 1. Stufe gegebenenfalls in Gegenwart eines Verdünnungsmittels und gegebenenfalls in Gegenwart eines Reaktionshilfsmittels umsetzt zu den Pyridylhydrazin-Derivaten der Formel (IV),

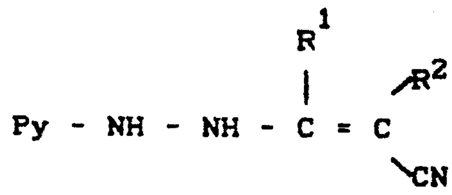

(IV)

in welcher

$R^1$, $R^2$ und Py die oben angegebene Bedeutung haben,

und diese in einer 2.Stufe, gegebenenfalls in Gegenwart eines Verdünnungsmittels und gegebenenfalls in Gegenwart eines Säurebindemittels cyclisiert,

oder man erhält

b) die erfindungsgemäßen 5-Amino-1-pyridyl-pyrazol-Derivate der Formel (Ib),

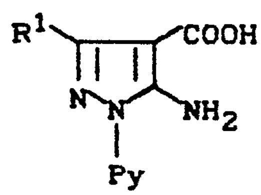

(Ib)

in welcher

$R^1$ und Py die oben angegebene Bedeutung haben,

wenn man 4-Alkoxycarbonyl-5-amino-pyrazole der Formel (Ir)

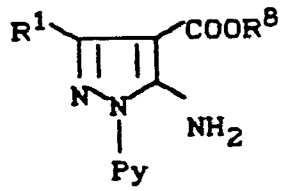

(Ir)

in welcher

$R^1$, $R^8$ und Py die oben angegebene Bedeutung haben,

an der Estergruppe in 4-Position des Pyrazolringes in allgemein üblicher Art und Weise, gegebenenfalls in Gegenwart eines Verdünnungsmittels und gegebenenfalls in Gegenwart eines Katalysators, verseift,

oder man erhält

c) die erfindungsgemäßen 5-Amino-1-pyridyl-pyrazol-Derivate der Formel (Ic),

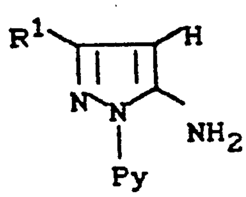

(Ic)

in welcher

$R^1$ und Py die oben angegebene Bedeutung haben,

wenn man 5-Amino-1-pyridyl-pyrazol-Derivate der Formel (Ib),

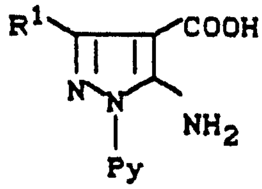

(Ib)

in welcher

$R^1$ und Py die oben angegebene Bedeutung haben,

in allgemein üblicher Weise, gegebenenfalls in Gegenwart eines Verdünnungsmittels und gegebenenfalls in Gegenwart eines Katalysators, decarboxyliert,

oder man erhält

d) die erfindungsgemäßen 5-Amino-1-pyridyl-pyrazol-Derivate der Formel (Id),

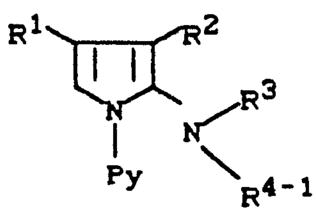

(Id)

in welcher

$R^{4-1}$ für Alkyl, für einen Rest $-\overset{\overset{X}{\|}}{C}-R^6$ oder für einen Rest $-S(O)_n-R^7$ steht und

$R^1$, $R^2$, $R^3$, $R^6$, $R^7$, X und n die oben angegebene Bedeutung haben,

wenn man 5-Amino-1-pyridyl-pyrazole der Formel (Is)

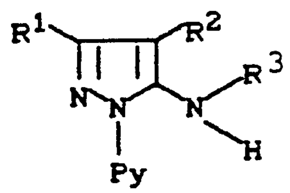

(Is)

in welcher

$R^1$, $R^2$, $R^3$ und Py die oben angegebene Bedeutung haben,

(d-α) mit Verbindungen der Formel (V),

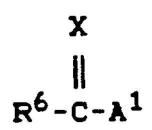

(V)

in welcher

$A^1$ für Halogen oder für einen Rest 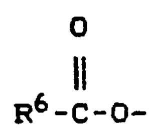 steht und

$R^6$ und X die oben angegebene Bedeutung haben, oder

(d-β) mit Verbindungen der Formel (Va),

$$R^7\text{-}S(O)_n\text{-}A^2 \qquad (Va)$$

in welcher

$A^2$ für Halogen steht und

$R^7$ und n die oben angegebene Bedeutung haben,

oder

(d-γ) mit Verbindungen der Formel (Vb),

$$R^8\text{-}A^3 \qquad (Vb)$$

in welcher

$R^8$ für Alkyl steht und

$A^3$ für Halogen, p-Toluolsulfonyloxy oder Alkoxysulfonyloxy steht,

gegebenenfalls in Gegenwart eines Verdünnungsmittels und gegebenenfalls in Gegenwart eines Säurebindemittels umsetzt,

oder man erhält

e) die erfindungsgemäßen 5-Amino-1-pyridyl-pyrazol-Derivate der Formel (Ie),

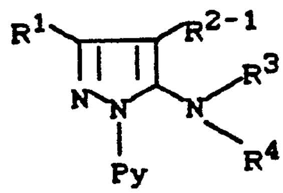

(Ie)

in welcher

$R^{2-1}$ für Halogen, Nitro, Nitroso, Formyl, Alkanoyl oder Aroyl steht und

$R^1$, $R^3$, $R^4$ und Py die oben angegebene Bedeutung haben,

wenn man 5-Amino-1-pyridyl-pyrazol-Derivate der Formel (It),

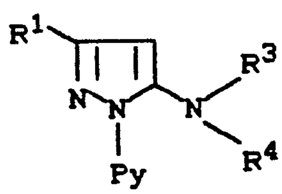

(It)

in welcher

$R^1$, $R^3$, $R^4$ und Py die oben angegebene Bedeutung haben,

mit elektrophilen Agenzien der Formel (VI),

$$R^{2-1}-A^4 \quad (VI)$$

in welcher

$A^4$ für eine elektronenanziehende Abgangsgruppe steht und

$R^{2-1}$ die oben angegebene Bedeutung hat,

oder mit anderen üblichen elektrophilen Agenzien gegebenenfalls in Gegenwart eines Verdünnungsmittels und gegebenenfalls in Gegenwart eines Katalysators oder Reaktionshilfsmittels in 4-Stellung substituiert,

oder man erhält

f) die erfindungsgemäßen 5-Amino-1-pyridyl-pyrazol-Derivate der Formel (If)

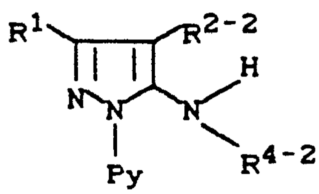

(If)

in welcher

$R^{2-2}$ für Halogen, Nitro oder Nitroso steht,

$R^{4-2}$ für Wasserstoff oder Alkyl steht und

$R^1$ und Py die oben angegebene Bedeutung haben,

wenn man 5-Acylamino-1-pyridyl-pyrazole der Formel (Iu)

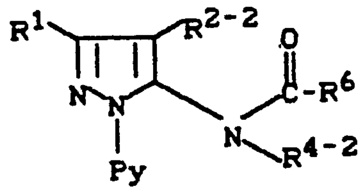

(Iu)

in welcher

$R^1$, $R^{2-2}$, $R^{4-2}$, $R^6$ und Py die oben angegebene Bedeutung haben,

gegebenenfalls in Gegenwart eines Verdünnungsmittels und gegebenenfalls in Gegenwart eines Katalysators an der Aminogruppe in 5-Position des Pyrazolringes deacyliert,

oder man erhält

g) die erfindungsgemäßen 5-Amino-1-pyridyl-pyrazol-Derivate der Formel (Ig)

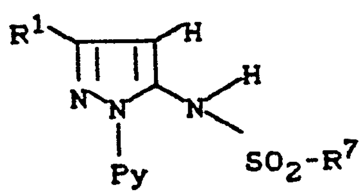

(Ig)

in welcher

$R^1$, $R^7$ und Py die oben angegebene Bedeutung haben,

wenn man 5-Bis-sulfonyl-amino-pyrazole der Formel (Iv)

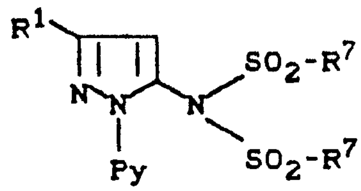

(Iv)

in welcher

$R^1$, $R^7$ und Py die oben angegebene Bedeutung haben,

mit Basen gegebenenfalls in Gegenwart eines Verdünnungsmittels spaltet,

oder man erhält

h) die erfindungsgemäßen 5-Amino-1-pyridyl-pyrazol-Derivate der Formel (Ih)

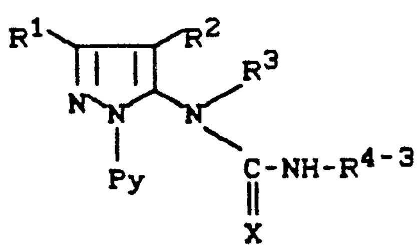

(Ih)

in welcher

$R^{4-3}$ für Alkyl oder für gegebenenfalls substituiertes Aryl steht und

$R^1$, $R^2$, $R^3$, X und Py die oben angegebene Bedeutung haben,

wenn man 5-Amino-1-pyridyl-pyrazole der Formel (Is),

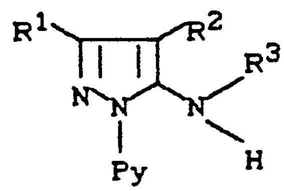

(Is)

in welcher

$R^1$, $R^2$, $R^3$ und Py die oben angegebene Bedeutung haben,

mit Iso(thio)cyanaten der Formel (VII),

$$R^{4-3} - N = C = X \qquad (VII)$$

in welcher

$R^{4-3}$ und X die oben angegebene Bedeutung haben,

gegebenenfalls in Gegenwart eines Verdünnungsmittels und gegebenenfalls in Gegenwart eines Säurebindemittels umsetzt,

oder man erhält

i) die erfindungsgemäßen 5-Amino-1-pyridyl-pyrazol-Derivate der Formel (Ii),

 

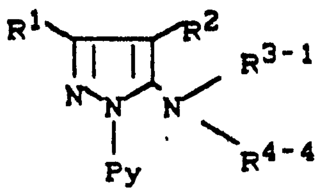

(Ii)

in welcher

$R^{4-4}$ für Alkyl steht,

$R^{3-1}$ für Wasserstoff oder Alkyl steht und

$R^1$, $R^2$ und Py die oben angegebene Bedeutung haben,

wenn man 5-Halogen-pyrazole der Formel (VIII),

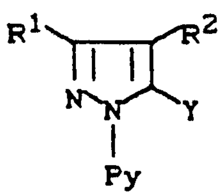

(VIII)

in welcher

Y für Halogen steht und

$R^1$, $R^2$ und Py die oben angegebene Bedeutung haben,

mit Aminen der Formel (IX),

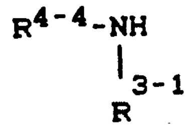

(IX)

in welcher

$R^{4-4}$ für Alkyl steht und

$R^{3-1}$ für Wasserstoff oder Alkyl steht,

gegebenenfalls in Gegenwart eines Verdünnungsmittels und gegebenenfalls in Gegenwart eines Säurebindemittels umsetzt,

oder man erhält

k) die erfindungsgemäßen 5-Amino-1-pyridyl-pyrazol-Derivate der Formel (Ik),

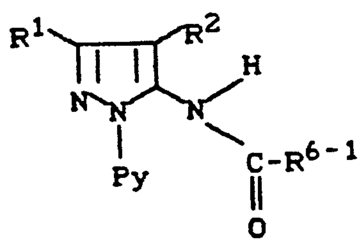

(Ik)

in welcher

$R^{6-1}$ für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht, und

$R^1$, $R^2$ und Py die oben angegebene Bedeutung haben,

wenn man (Bis)Carbamate der Formel (Iw),

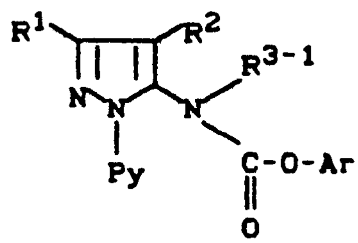

(Iw)

in welcher

$R^{3-1}$ für Wasserstoff oder für einen Rest $-\overset{O}{\overset{\|}{C}}-O-Ar$ steht, wobei

Ar für gegebenenfalls substituiertes Aryl steht und

$R^1$, $R^2$ und Py die oben angegebene Bedeutung haben,

mit Verbindungen der Formel (X),

$$R^{6-1} - H \qquad (X)$$

in welcher

$R^{6-1}$ die oben angegebene Bedeutung hat,

gegebenenfalls in Gegenwart eines Verdünnungsmittels und gegebenenfalls in Gegenwart eines basischen Katalysators umsetzt,

oder man erhält

l) Salze von erfindungsgemäßen 5-Sulfonamido-pyrazol-Derivaten der Formel (Ix),

wenn man 5-Sulfonamido-pyrazole der Formel (Ix),

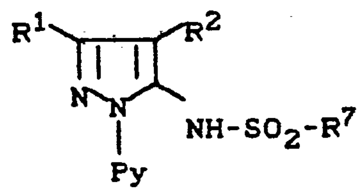

(Ix)

in welcher

$R^1$, $R^2$, $R^7$ und Py die oben angegebene Bedeutung haben,

entweder mit Salzen der Formel (XI),

$$M^{\oplus} - G^{\ominus} \quad \text{(XI)}$$

in welcher

$M^{\oplus}$ für ein Äquivalent eines anorganischen oder organischen Kations steht und

$G^{\ominus}$ für ein Äquivalent eines geeigneten Gegenions steht,

oder mit primären, sekundären oder tertiären Aminen gegebenenfalls in Gegenwart eines Verdünnungsmittels umsetzt.

Schließlich wurde gefunden, daß die neuen 5-Amino-1-pyridyl-pyrazole der allgemeinen Formel (I) herbizide, insbesondere auch selektiv-herbizide Eigenschaften besitzen.

Überraschenderweise zeigen die erfindungsgemäßen 5-Amino-1-pyridyl-pyrazole der allgemeinen Formel (I) neben einer deutlich verbesserten allgemein-herbiziden Wirksamkeit gegenüber Schadpflanzen auch eine erheblich verbesserte Verträglichkeit gegenüber wichtigen Kulturpflanzen als die aus dem Stand der Technik bekannten 5-Amino-1-aryl-pyrazole, wie beispielsweise das 4-Cyano-5-propionamido-1-(2,3,4-trichlorphenyl)-pyrazol, welche chemisch und wirkungsgemäß naheliegende Verbindungen sind.

Die erfindungsgemäßen 5-Amino-1-pyridyl-pyrazole sind durch die Formel (I) allgemein definiert. Bevorzugt sind Verbindungen der Formel (I), bei welchen

$R^1$ für Wasserstoff oder für geradkettiges oder verzweigtes Alkyl mit 1 bis 8 Kohlenstoffatomen steht,

$R^2$ für Wasserstoff, Nitro, Nitroso, Fluor, Chlor, Brom, Iod oder für einen Rest $-\underset{\underset{O}{\|}}{C}-R^5$ steht, wobei

$R^5$ für Wasserstoff, Hydroxy, geradkettiges oder verzweigtes Alkyl mit 1 bis 12 Kohlenstoffatomen, für jeweils geradkettiges oder verzweigtes Alkenyl, Alkinyl, Alkoxyalkyl, Alkylthioalkyl, Alkoxy, Alkylthio, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht, außerdem für gegebenenfalls einfach oder mehrfach, gleich oder verschieden durch Halogen, $C_1$-$C_4$-Alkyl oder $C_1$-$C_4$-Halogenalkyl substituiertes Cycloalkyl mit 3 bis 7 Kohlenstoffatomen, sowie für jeweils gegebenenfalls einfach oder mehrfach, gleich oder verschieden substituiertes Phenyl, Phenoxy, Phenylthio oder Phenylamino steht, wobei als Phenylsubstituenten jeweils in Frage kommen: Halogen, jeweils geradkettiges oder verzweigtes Alkyl, Alkoxy mit jeweils 1 bis 4 Kohlenstoffatomen und Halogenalkyl mit 1 bis 4 Kohlenstoffatomen und 1 bis 9 gleichen oder verschiedenen Halogenatomen,

$R^3$ für Wasserstoff, für einen Rest $-\underset{\underset{X}{\|}}{C}-R^6$ oder für einen Rest $-S(O)_n-R^7$ steht,

$R^4$ für Wasserstoff, für einen Rest $-\underset{\underset{X}{\|}}{C}-R^6$ oder für einen Rest $-S(O)_n-R^7$ steht, für geradkettiges oder

verzweigtes Alkyl mit 1 bis 6 Kohlenstoffatomen steht, oder für den Fall, daß $R^3$ für einen Rest $-SO_2-R^7$ steht auch für ein salzartig gebundenes Äquivalent eines Alkali- oder Erdalkali- oder Übergangsmetallkations oder für ein gegebenenfalls durch $C_1$-$C_4$-Alkyl oder Phenyl substituiertes Ammoniumion steht, wobei

$R^6$ für Wasserstoff, für geradkettiges oder verzweigtes Alkyl mit 1 bis 12 Kohlenstoffatomen, für jeweils geradkettiges oder verzweigtes Alkenyl oder Alkinyl, mit jeweils 2 bis 4 Kohlenstoffatomen, für jeweils geradkettiges oder verzweigtes Alkoxyalkyl, Alkylthioalkyl, Alkoxy, Alkylthio, Alkylamino, Dialkylamino oder Halogenalkyl mit jeweils 1 bis 4 Kohlenstoffatomen in den einzelnen Alkylteilen und im Fall des Halogenalkyl mit bis 9 gleichen oder verschiedenen Halogenatomen steht, außerdem für gegebenenfalls einfach oder mehrfach, gleich oder verschieden durch Halogen, $C_1$-$C_4$-Alkyl oder $C_1$-$C_4$-Halogenalkyl substituiertes Cycloalkyl mit 3 bis 7 Kohlenstoffatomen, sowie für jeweils gegebenenfalls einfach oder mehrfach, gleich oder verschieden substituiertes Phenyl, Phenoxy, Phenylthio oder Phenylamino steht, wobei als Phenylsubstituenten jeweils in Frage kommen: Halogen, jeweils geradkettiges oder verzweigtes Alkyl, Alkoxy mit jeweils 1 bis 4 Kohlenstoffatomen und Halogenalkyl mit 1 bis 4 Kohlenstoffatomen und 1 bis 9 gleichen oder verschiedenen Halogenatomen,

$R^7$ für jeweils geradkettiges oder verzweigtes Alkyl oder Halogenalkyl mit jeweils 1 bis 4 Kohlenstoffatomen und gegegebenenfalls 1 bis 9 gleichen oder verschiedenen Halogenatomen oder für gegebenenfalls einfach oder mehrfach, gleich oder verschieden substituiertes Phenyl steht, wobei als Phenylsubstituenten in Frage kommen: Halogen, jeweils geradkettiges oder verzweigtes Alkyl, Alkoxy mit jeweils 1 bis 4 Kohlenstoffatomen und Halogenalkyl mit 1 bis 4 Kohlenstoffatomen und 1 bis 9 gleichen oder verschiedenen Halogenatomen,

X für Sauerstoff oder Schwefel steht,

n für eine Zahl 0, 1 oder 2 steht und

Py für jeweils einfach oder mehrfach, gleich oder verschieden substituiertes 2-Pyridyl, 3-Pyridyl oder 4-Pyridyl steht, wobei als Substituenten in Frage kommen: Cyano, Nitro, Halogen, jeweils geradkettiges oder verzweigtes Alkyl, Alkoxy oder Alkoxycarbonyl mit jeweils 1 bis 4 Kohlenstoffatomen im Alkylteil, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_m-R^9$, wobei

$R^9$ für Amino, für jeweils geradkettiges oder verzweigtes Alkyl, Alkylamino oder Dialkylamino mit jeweils 1 bis 4 Kohlenstoffatomen in den einzelnen Alkylteilen oder für Halogenalkyl mit 1 bis 4 Kohlenstoffatomen und mit 1 bis 9 gleichen oder verschiedenen Halogenatomen steht und

m für eine Zahl 0, 1 oder 2 steht.

Besonders bevorzugt sind Verbindungen der Formel (I), bei welchen

$R^1$ für Wasserstoff, Methyl, Ethyl, n- oder i-Propyl, n-, i-, s- oder t-Butyl steht,

$R^2$ für Wasserstoff, Nitro, Nitroso, Fluor, Chlor, Brom, Iod oder für einen Rest $-\underset{\underset{O}{\|}}{C}-R^5$ steht, wobei

$R^5$ für Wasserstoff, Hydroxy, Methyl, Ethyl, n- oder i-Propyl, n-, i-, s- oder t-Butyl, n- oder i-Pentyl, Vinyl, Allyl, Propargyl, Butenyl, Methoxymethyl, Ethoxymethyl, Methoxyethyl, Ethoxyethyl, Methylthiomethyl, Methoxy, Ethoxy, Methylthio, Ethylthio, Methylamino, Ethylamino, Dimethylamino, Diethylamino, Trifluormethyl, Trichlorethyl, Dichlorfluorethyl, Difluorchlorethyl, Chlormethyl, Iodmethyl, Brommethyl, Dichlormethyl, 1-Chlorethyl, 2-Chlorethyl, 2-Bromethyl, 2-Chlorpropyl, Heptafluor-n-propyl, für jeweils gegebenenfalls ein- bis vierfach, gleich oder verschieden durch Fluor, Chlor, Brom, Methyl oder Trifluormethyl substituiertes Cyclopropyl, Cyclopentyl oder Cyclohexyl, für jeweils gegebenenfalls ein- bis dreifach, gleich oder verschieden durch Methyl, Methoxy, Chlor oder

Trifluormethyl substituiertes Phenyl, Phenoxy, Phenylthio oder Phenylamino steht,

$R^3$ für Wasserstoff, für einen Rest $-\underset{\underset{X}{\|}}{C}-R^6$ oder für einen Rest $-S(O)_n-R^7$ steht,

$R^4$ für Wasserstoff, für einen Rest $-\overset{\overset{X}{\|}}{C}-R^6$ oder für einen Rest $-S(O)_n-R^7$ steht, für Methyl, Ethyl, n- oder i-Propyl, n-, i-, s- oder t-Butyl steht oder für den Fall, daß $R^3$ für einen Rest $-SO_2-R^7$ steht, auch für ein salzartig gebundenes Äquivalent eines Natrium-, Kalium-, Magnesium-, Calcium-, Barium-, Kupfer-, Zink-, Mangan-, Zinn-, Eisen-, Cobalt-oder Nickelions steht, oder für ein gegebenenfalls ein- bis dreifach, gleich oder verschieden durch Methyl, Ethyl, n- oder i-Propyl, n-, i-, s- oder t-Butyl oder Phenyl substituiertes Ammoniumion steht, wobei

$R^6$ für Wasserstoff, Methyl, Ethyl, n- oder i-Propyl, n-, i-, s- oder t-Butyl, n- oder i-Pentyl, n- oder i-Hexyl, n- oder i-Heptyl, Vinyl, Allyl, Propargyl, Butenyl, Methoxymethyl, Ethoxymethyl, Methoxyethyl, Ethoxyethyl, Methylthiomethyl, Methoxy, Ethoxy, Methylthio, Ethylthio, Methylamino, Ethylamino, Dimethylamino, Diethylamino, Trifluormethyl, Trichlorethyl, Dichlorfluorethyl, Difluorchlorethyl, Chlormethyl, Iodmethyl, Brommethyl, Dichlormethyl, 1-Chlorethyl, 2-Chlorethyl, 2-Bromethyl, 3-Chlorpropyl, Heptafluor-n-propyl, für jeweils gegebenenfalls ein- bis vierfach, gleich oder verschieden

durch Fluor, Chlor, Brom, Methyl oder Trifluormethyl substituiertes Cyclopropyl, Cyclopentyl oder Cyclohexyl, für jeweils gegebenenfalls ein- bis dreifach, gleich oder verschieden durch Methyl, Methoxy, Chlor oder Trifluormethyl substituiertes Phenyl, Phenoxy, Phenylthio oder Phenylamino steht,

$R^7$ für Methyl, Ethyl, n- oder i-Propyl, n-, i-, s- oder t-Butyl, Chlormethyl, Dichlormethyl, Trichlormethyl, Trifluormethyl oder für gegebenenfalls ein- bis dreifach, gleich oder verschieden durch Methyl, Methoxy, Chlor oder Trifluormethyl substituiertes Phenyl steht,

X für Sauerstoff oder Schwefel steht,

n für eine Zahl 0, 1 oder 2 steht und

Py für jeweils ein- bis vierfach, gleich oder verschieden substituiertes 2-Pyridyl oder 4-Pyridyl steht, wobei als Substituenten in Frage kommen: Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl,

Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^9$,

wobei

$R^9$ für Amino, Methylamino, Ethylamino, Dimethylamino, Diethylamino, Fluordichlormethyl, Difluormethyl, Tetrafluorethyl, Trifluorchlorethyl, Trifluormethyl, Methyl oder Ethyl steht und

m für eine Zahl 0, 1 oder 2 steht.

Im einzelnen seien außer den bei den Herstellungsbeispielen genannten Verbindungen die folgenden 5-Amino-1-pyridyl-pyrazole der allgemeinen Formel (I) genannt:

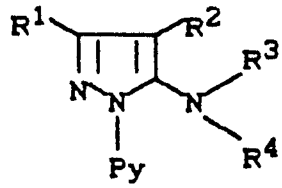

(I)

Tabelle 1:

| $R^1$ | $R^2$ | $R^3$ | $R^4$ | Py |
|---|---|---|---|---|
| H | $NO_2$ | H | H | 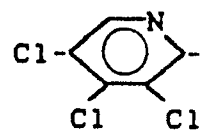 |
| H | $NO_2$ | $-CO-C_2H_5$ | H | 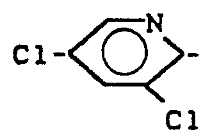 |
| H | Cl | H | H | 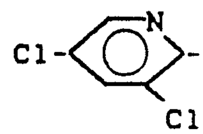 |
| H | Cl | $-CO-C_2H_5$ | H | 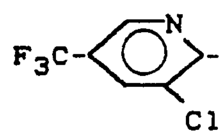 |
| H | $NO_2$ | $-CO-C_2H_5$ | $CH_3$ | 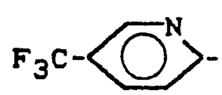 |
| H | $NO_2$ | H | H | 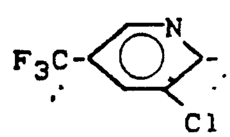 |

Tabelle 1 (Fortsetzung)

| $R^1$ | $R^2$ | $R^3$ | $R^4$ | Py |
| --- | --- | --- | --- | --- |
| H | $NO_2$ | $-CO-C_2H_5$ | H | 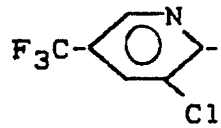 |
| H | $-CO-OC_2H_5$ | H | H | 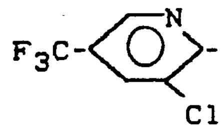 |
| H | $-CO-OC_2H_5$ | H | H | 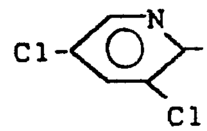 |
| H | $NO_2$ | $-CO-CHCl_2$ | H | 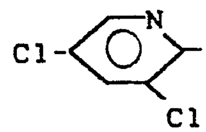 |
| H | $NO_2$ | $-CO-CHCl_2$ | H | 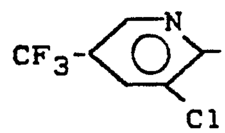 |
| H | $NO_2$ | $-C_4H_9-n$ | H | 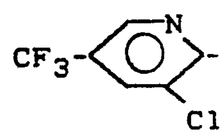 |
| H | $NO_2$ | H | H | 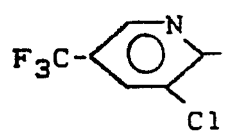 |

Tabelle 1 (Fortsetzung)

| $R^1$ | $R^2$ | $R^3$ | $R^4$ | Py |
|---|---|---|---|---|
| $CH_3$ | $NO_2$ | H | H | 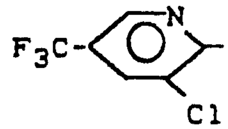 |
| H | $NO_2$ | $-CO-OCH_3$ | H | 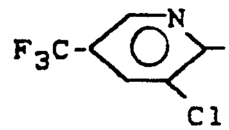 |
| H | $NO_2$ | $-CO-NH-C_3H_7-i$ | H | 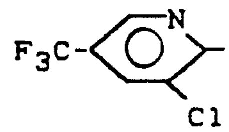 |
| H | $NO_2$ | -CO-O- 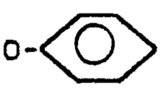 | H | 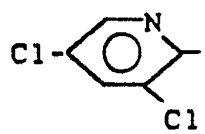 |
| H | $NO_2$ | $-CO-NH-CH_3$ | H | 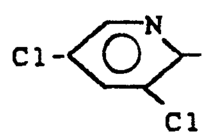 |
| H | $NO_2$ | $-SO_2-CH_3$ | H | 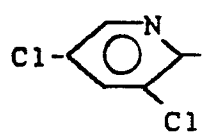 |
| H | $NO_2$ | $-SO_2-CH_3$ | $-SO_2-CH_3$ | 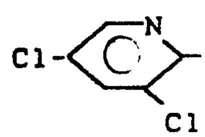 |

Tabelle 1 (Fortsetzung)

| $R^1$ | $R^2$ | $R^3$ | $R^4$ | Py |
|---|---|---|---|---|
| H | $NO_2$ | $-CO-C_2H_5$ | $-C_3H_7-n$ | 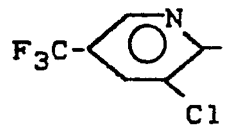 |
| H | $NO_2$ | H | H | 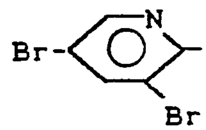 |
| H | $NO_2$ | H | H | 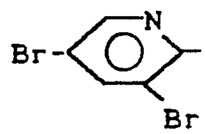 |
| H | $-CO-OC_2H_5$ | H | H | 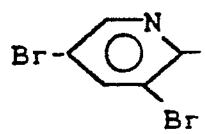 |
| $CH_3$ | $-CO-OC_2H_5$ | H | H | 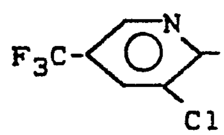 |
| $CH_3$ | $NO_2$ | $-CO-C_2H_5$ | H | 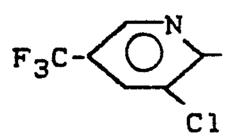 |

Verwendet man beispielsweise N-(3,5-Dichlorpyrid-2-yl)-hydrazin und Ethoxymethylenmalonsäuremonoethylesternitril als Ausgangsstoffe, so läßt sich der Reaktionsablauf des erfindungsgemäßen Verfahrens (a) durch das folgende Formelschema darstellen:

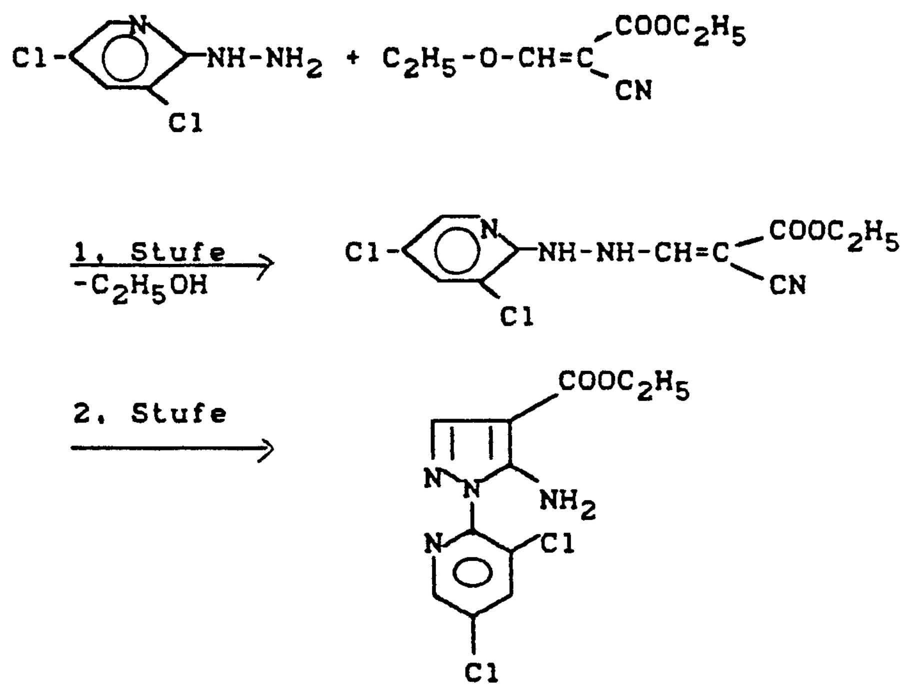


Verwendet man beispielsweise 5-Amino-1-(3,5-dichlorpyrid-2-yl)-4-ethoxycarbonyl-pyrazol als Ausgangsstoff, so läßt sich der Reaktionsablauf des erfindungsgemäßen Verfahrens (b) durch das folgende Formelschema darstellen:

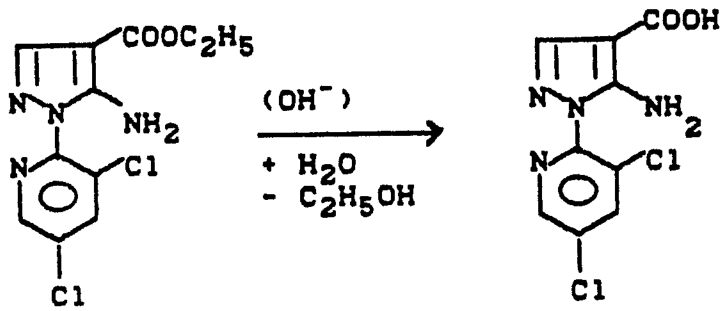


Verwendet man beispielsweise 5-Amino-1-(3,5-dichlorpyrid-2-yl)-pyrazol-4-carbonsäure als Ausgangsstoff , so läßt sich der Reaktionsablauf des erfindungsgemäßen Verfahrens (c) durch das folgende Formelschema darstellen:

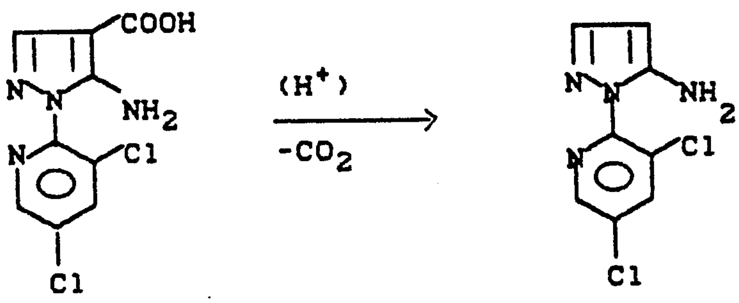


Verwendet man beispielsweise 5-Amino-1-(3,5-dichlorpyrid-2-yl)-pyrazol und Propionylchlorid als Ausgangsstoffe, so läßt sich der Reaktionsablauf des erfindungsgemäßen Verfahrens (d) durch das folgende Formelschema darstellen:

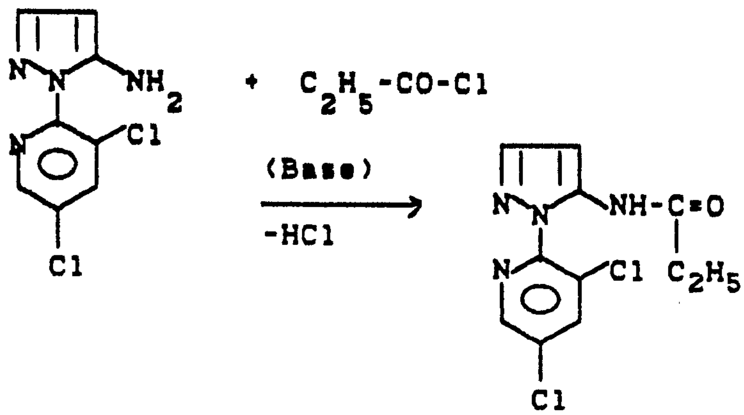


Verwendet man beispielsweise 1-(3,5-Dichlorpyrid-2-yl)-5-propionamido-pyrazol und Salpetersäure als Ausgangsstoffe, so läßt sich der Reaktionsablauf des erfindungsgemäßen Verfahrens (e) durch das folgende Formelschema darstellen:

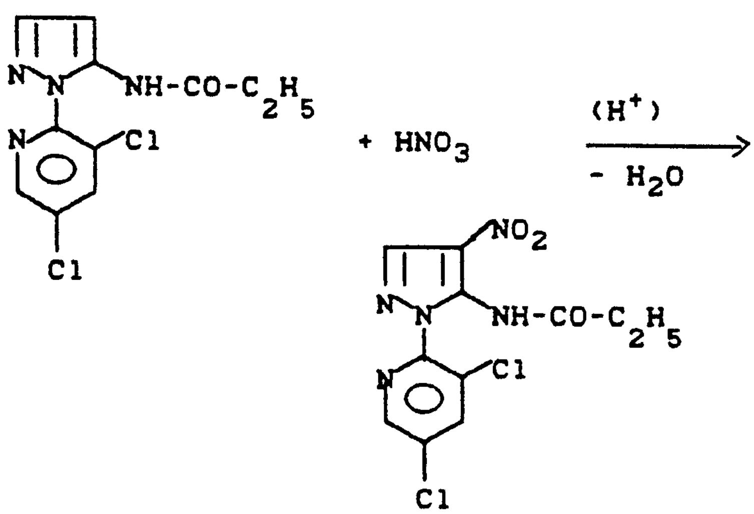

Verwendet man beispielsweise 1-(3,5-Dichlor-pyrid-2-yl)-4-nitro-5-propionamido-pyrazol als Ausgangsstoff, so läßt sich der Reaktionsablauf des erfindungsgemäßen Verfahrens (f) durch das folgende Formelschema darstellen:

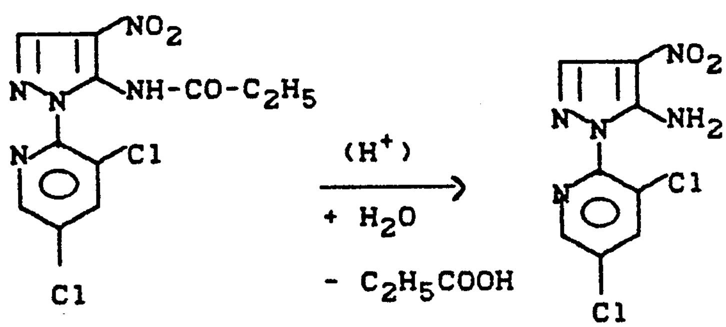


Verwendet man beispielsweise 5-[N,N-Bis(methansulfon)-amido]-1-(3,5-dichlorpyrid-2-yl)-pyrazol und Ammoniak als Ausgangsstoffe, so läßt sich der Reaktionsablauf des erfindungsgemäßen Verfahrens (g) durch das folgende Formelschema darstellen:

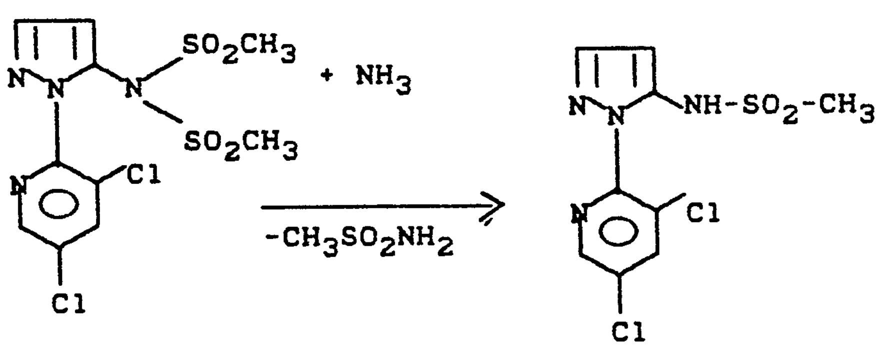

Verwendet man beispielsweise 5-Amino-1-(3,5-dichlor-pyrid-2-yl)-pyrazol und Methylisocyanat als Ausgangsstoffe, so läßt sich der Reaktionsablauf des erfindungsgemäßen Verfahrens (h) durch das folgende Formelschema darstellen:

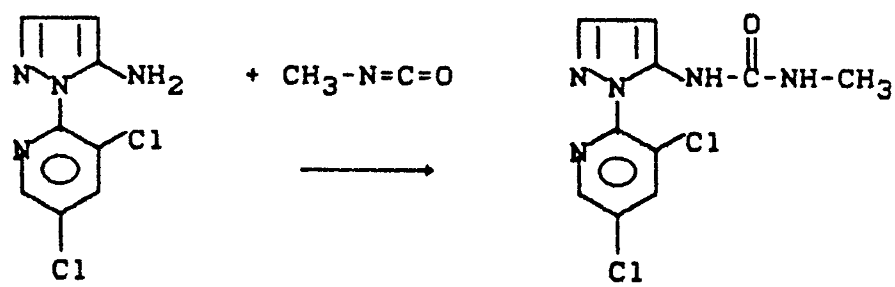

Verwendet man beispielsweise 5-Brom-4-nitro-1-(3,5-dichlorpyrid-2-yl)-pyrazol und Isopropylamin als Ausgangsstoffe, so läßt sich der Reaktionsablauf des erfindungsgemäßen Verfahrens (i) durch das folgende Formelschema darstellen:

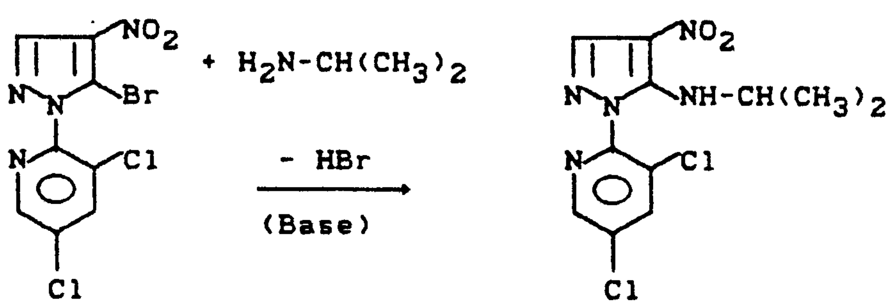


Verwendet man beispielsweise 5-Phenoxycarbonylamino-1-(3,5-dichlorpyrid-2-yl)-pyrazol und Methanol als Ausgangsstoffe, so läßt sich das erfindungsgemäße Verfahren (k) durch das folgende Formelschema darstellen:

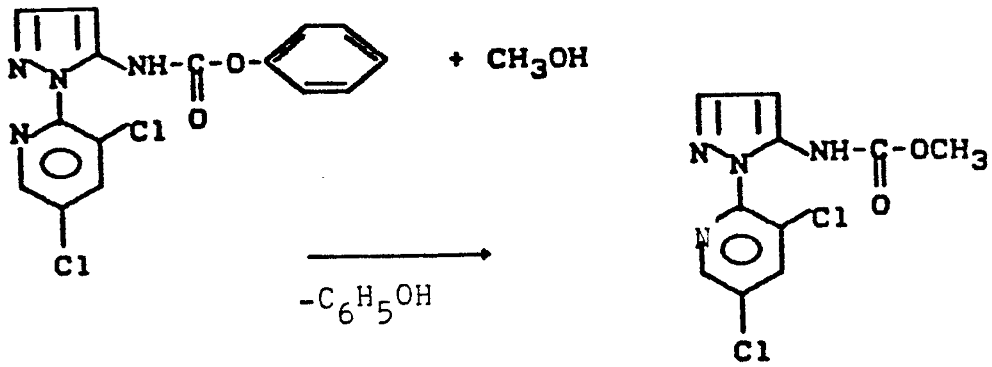


Verwendet man beispielsweise 1-(3,5-Dichlorpyrid-2-yl)-5-methansulfonamido-pyrazol und Isopropylamin als Ausgangsstoffe, so läßt sich der Reaktionsablauf des erfindungsgemäßen Verfahrens (1) durch das folgende Formelschema darstellen:

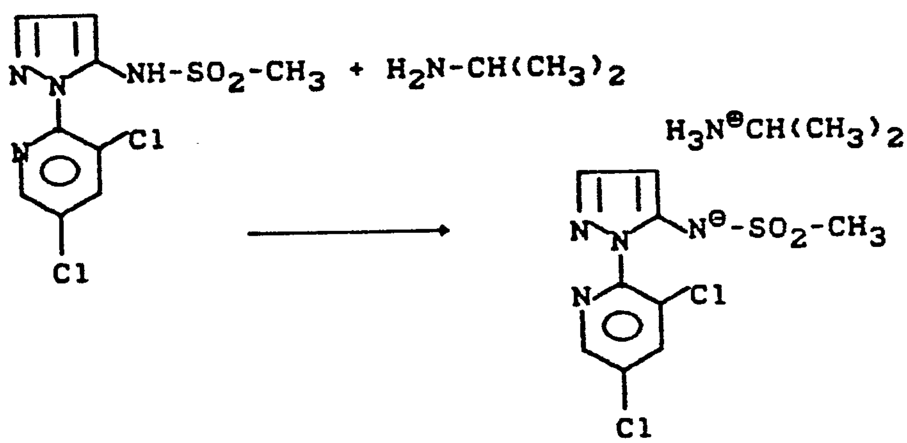

Die zur Durchführung des erfindungsgemäßen Verfahrens (a) als Ausgangsstoffe benötigten Pyridylhydrazine sind durch die Formel (II) allgemein definiert. In dieser Formel (II) steht Py vorzugsweise für diejenigen Reste,

die bereits im Zusammenhang mit der Beschreibung der erfindungsgemäßen Stoffe der Formel (I) als bevorzugt für diesen Substituenten genannt wurden.

Die Pyridylhydrazine der Formel (II) sind bekannt (vgl.z.B. US-PS 4.127.575; US-PS 3.609.158; DE-OS 25 58 399; J.Chem.Soc.C., 1971, 167-174) oder lassen sich nach prinzipiell bekannten Verfahren in einfacher analoger Weise herstellen, z.B. wenn man Halogenpyridine der Formel (XII),

Py - Hal (XII)

in welcher

Py die oben angegebene Bedeutung hat und

Hal für Halogen, insbesondere für Fluor, Chlor oder Brom steht,

mit Hydrazinhydrat gegebenenfalls in Gegenwart eines Verdünnungsmittels wie beispielsweise Pyridin oder Dioxan bei Temperaturen zwischen 0°C und 150°C umsetzt, oder wenn man beispielsweise Aminopyridine der Formel (XIII),

$$Py - NH_2 \quad (XIII)$$

in welcher

Py die oben angegebene Bedeutung hat,

in bekannter Weise, z.B. mit Natriumnitrit in Gegenwart einer Säure, wie beispielsweise Schwefelsäure, diazotiert und anschließend ebenfalls in bekannter Weise die so erhältlichen Diazoniumsalze beispielsweise mit Zinn-II- chlorid in Gegenwart einer Säure, wie beispielsweise Salzsäure, bei Temperaturen zwischen -20°C und +80°C reduziert.

Die zur Durchführung des erfindungsgemäßen Verfahrens (a) weiterhin als Ausgangsstoffe benötigten Acrylnitril-Derivate sind durch die Formel (III) allgemein definiert. In dieser Formel (III) stehen $R^1$ und $R^2$ vorzugsweise für diejenigen Reste, die bereits im Zusammenhang mit der Beschreibung der erfindungsgemäßen Stoffe der Formel (I) als bevorzugt für $R^1$ und $R^2$ genannt wurden. A steht vorzugsweise für Chlor, Brom, Hydroxy, Methoxy, Ethoxy oder Dimethylamino.

Die Acrylnitril-Derivate der Formel (III) sind bekannt (vgl. DE-OS 31 29 429, DE-OS 32 06 878, EP 34 945; J.Chem.Soc.D 1255; 1970, Can.J.Chem. 48, 2104-2109 (1970); J.Heterocyclic Chem. 19, 1267-1273 (1982); Can. J.Chem. 51, 1239-1244 (1973)) oder können nach bekannten Verfahren in einfacher analoger Weise erhalten werden.

Die Halogenpyridine der Formel (XII) und die Aminopyridine der Formel (XIII) sind allgemein bekannte Verbindungen der organischen Chemie.

Die zur Durchführung des erfindungsgemäßen Verfahrens (b) als Ausgangsstoffe benötigten 4-Alkoxycarbonyl-5-amino-pyrazole sind durch die Formel (Ir) allgemein definiert. In der Formel (Ir) stehen $R^1$ und Py vorzugsweise für diejenigen Reste, die bereits im Zusammenhang mit der Beschreibung der erfindungsgemäßen Stoffe der Formel (I) als bevorzugt für diese Substituenten genannt wurden. $R^8$ steht vorzugsweise für geradkettiges oder verzweigtes Alkyl mit 1 bis 4 Kohlenstoffatomen, insbesondere für Methyl oder Ethyl.

Die 4-Alkoxycarbonyl-5-amino-pyrazole der Formel (Ir) sind erfindungsgemäße Verbindungen und erhältlich mit Hilfe des erfindungsgemäßen Verfahrens (a).

Die zur Durchführung des erfindungsgemäßen Verfahrens (c) als Ausgangsstoffe benötigten 5-Amino-1-pyridyl-pyrazol-Derivate sind durch die Formel (Ib) allgemein definiert. In dieser Formel (Ib) stehen $R^1$ und Py vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Amino-1-pyridyl-pyrazol-Derivate der Formel (Ib) sind erfindungsgemäße Verbindungen und erhältlich mit Hilfe des erfindungsgemäßen Verfahrens (b).

Die zur Durchführung des erfindungsgemäßen Verfahrens (d) als Ausgangsstoffe benötigten 5-Amino-1-pyridyl-pyrazole sind durch die Formel (Is) allgemein definiert.

In dieser Formel (Is) stehen $R^1$, $R^2$, $R^3$ und Py vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Amino-1-pyridyl-pyrazole der Formel (Is) sind erfindungsgemäße Verbindungen.

5-Amino-1-pyridyl-pyrazole der Formel (Is), bei welchen $R^3$ für Wasserstoff steht, sind erhältlich mit Hilfe der erfindungsgemäßen Verfahren (a), (b), (c), (e) oder (f). 5-Amino-1-pyridyl-pyrazole der Formel (Is), in welchen $R^3$ verschieden von Wasserstoff ist, sind erhältlich mit Hilfe der erfindungsgemäßen Verfahren (e), (f), (g) oder (h).

Außerdem können 5-Amino-1-pyridyl-pyrazole der Formel (Id), welche beispielsweise nach dem erfindungsgemäßen Verfahren (d-α) hergestellt werden, im erfindungsgemäßen Verfahren (d-γ) als Ausgangsstoffe eingesetzt werden.

Setzt man die mit Hilfe der erfindungdsgemäßen Verfahren (d-α), (d-β) oder (d-γ) erhaltenen mono-alkylierten, -acylierten, -sulfenylierten, -sulfinylierten oder -sulfonylierten Verbindungen erneut nach einem dieser Verfahren um, erhält man die entsprechenden disubstituierten Verbindungen.

Die zur Durchführung des erfindungsgemäßen Verfahrens (d) weiterhin benötigten Verbindungen sind durch die Formeln (V), (Va) und (Vb) allgemein definiert. In den Formeln (V), (Va) und (Vb) stehen $R^8$ vorzugsweise für geradkettiges oder verzweigtes Alkyl mit 1 bis 6 Kohlenstoffatomen und $R^6$, $R^7$, X und n vorzugsweise für diejenigen Reste, die bereits im Zusammenhang mit der Beschreibung der erfindungsgemäßen Stoffe der Formel (I) als bevorzugt für diese Substituenten genannt wurden, $A^1$ steht vorzugsweise für Chlor oder Brom oder für einen Rest $R^6-\overset{\overset{O}{\|}}{C}-O$, $A^2$ steht bevorzugt für Chlor oder Brom und $A^3$ steht bevorzugt für Chlor, Brom, Jod, p-Toluolsulfonyloxy oder Methoxysulfonyloxy.

Die Verbindungen der Formeln (V), (Va) und (Vb) sind allgemein bekannte Verbindungen der organischen Chemie.

Die zur Durchführung des erfindungsgemäßen Verfahrens (e) als Ausgangsstoffe benötigten 5-Amino-1-pyridyl-pyrazole sind durch die Formel (It) allgemein definiert. In dieser Formel (It) stehen $R^1$, $R^3$, $R^4$ und Py vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Amino-1-pyridyl-pyrazole der Formel (It) sind erfindungsgemäßeVerbindungen und erhältlich mit Hilfe der erfindungsgemäßen Verfahren (a), (c), (d), (g), (i) oder (k).

Die zur Durchführung des erfindungsgemäßen Verfahrens (e) weiterhin als Ausgangsstoffe benötigten elektrophilen Agenzien sind durch die Formel (VI) allgemein definiert. In dieser Formel (VI) steht $R^{2-1}$ vorzugsweise für Chlor, Brom, Nitroso, Nitro, für Formyl, Alkanoyl mit 1 bis 6 Kohlenstoffatomen im Alkylteil oder für gegebenenfalls einfach oder mehrfach, gleich oder verschieden substituiertes Benzoyl, wobei als Substituenten in Frage kommen: Halogen, insbesondere Fluor, Chlor oder Brom, jeweils geradkettiges oder verzweigtes Alkyl, Alkoxy mit jeweils 1 bis 4 Kohlenstoffatomen, insbesondere Methyl oder Methoxy, Halogenalkyl mit 1 bis 4 Kohlenstoffatomen und 1 bis 9 gleichen oder verschiedenen Halogenatomen, insbesondere Trifluormethyl.

$A^4$ steht vorzugsweise für Halogen, insbesondere für Chlor oder Brom, für Hydroxy, für Alkyl- oder Arylsulfonyloxy, für Alkanoyloxy oder Aroyloxy. Weiterhin verwendbare elektrophile Reagenzien sind Sulfurylchlorid, Phosphoroxychlorid/Dimethylformamid, Nitriersäure und andere üblicherweise zu elektrophilen Substitutionen verwendbare Stoffe.

Die elektrophilen Agenzien der Formel (VI) sind ebenso wie die weiteren üblichen elektrophilen Reagenzien allgemein bekannte Verbindungen.

Die zur Durchführung des erfindungsgemäßen Verfahrens (f) als Ausgangsstoffe benötigten 5-Acylamino-1-pyridylpyrazole sind durch die Formel (Iu) allgemein definiert.

In dieser Formel (Iu) stehen $R^1$, $R^6$ und Py vorzugsweise für diejenigen Reste, die bereits im Zusammenhang mit der Beschreibung der erfindungsgemäßen Stoffe der Formel (I) als bevorzugt für diese Substituenten genannt wurden.

$R^{2-2}$ steht vorzugsweise für Nitro, Nitroso, Fluor, Chlor, Brom oder Iod, $R^{4-2}$ steht vorzugsweise für Wasserstoff oder für geradkettiges oder verzweigtes Alkyl mit 1 bis 4 Kohlenstoffatomen, insbesondere für Methyl oder Ethyl.

Die 5-Acylamino-1-pyridyl-pyrazole der Formel (Iu) sind erfindungsgemäße Verbindungen und erhältlich mit Hilfe der erfindungsgemäßen Verfahren (d) oder (e).

Die zur Durchführung des erfindungsgemäßen Verfahrens (g) als Ausgangsstoffe benötigten 5-Bis-sulfonyl-amino-pyrazole sind durch die Formel (Iv) allgemein definiert. In dieser Formel (Iv) stehen $R^1$, $R^7$ und Py vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Bis-sulfonyl-amino-pyrazole der Formel (Iv) sind erfindungsgemäße Verbindungen und erhältlich mit Hilfe des erfindungsgemäßen Verfahrens (d).

Die zur Durchführung des erfindungsgemäßen Verfahrens (h) als Ausgangsstoffe benötigten 5-Amino-1-pyridyl-pyrazole sind durch die Formel (Is) allgemein definiert.

In dieser Formel (Is) haben $R^1$, $R^2$, $R^3$ und Py vorzugsweise für diejenigen Bedeutungen, die bereits im Zusammenhang mit der Beschreibung der erfindungsgemäßen Stoffe der Formel (I) als bevorzugt für diese Substituenten genannt werden.

Die 5-Amino-1-pyridyl-pyrazole der Formel (Is) sind erfindungsgemäße Verbindungen. Verbindungen der Formel (Is), bei welchen $R^3$ für Wasserstoff steht, sind erhältlich mit Hilfe der erfindungsgemäßen Verfahren (a), (b), (c), (e) oder (f).

Verbindungen der Formel (Is), in welchen $R^3$ verschieden von Wasserstoff ist, sind erhältlich mit Hilfe der erfindungsgemäßen Verfahren (d), (e), (f), (g) oder (h).

Die zur Durchführung des erfindungsgemäßen Verfahrens (h) weiterhin als Ausgangsstoffe benötigten Iso(thio)-cyanate sind durch die Formel (VII) allgemein definiert. In dieser Formel (VII) steht X für Sauerstoff oder Schwefel und $R^{4-3}$ vorzugsweise für geradkettiges oder verzweigtes Alkyl mit 1 bis 4 Kohlenstoffatomen oder für gegebenenfalls ein- bis dreifach, gleich oder verschieden substituiertes Phenyl, wobei als Substituenten in Frage kommen: Halogen, jeweils geradkettiges oder verzweigtes Alkyl oder Alkoxy mit jeweils 1 bis 4 Kohlenstoffatomen oder Halogenalkyl mit 1 bis 4 Kohlenstoffatomen und 1 bis 9 gleichen oder verschiedenen Halogenatomen. $R^{4-3}$ steht insbesondere für Methyl, Ethyl oder für gegebenenfalls ein- bis dreifach, gleich oder verschieden durch Fluor, Chlor, Methyl, Methoxy oder Trifluormethyl substituiertes Phenyl.

Die Iso(thio)cyanate der Formel (VII) sind allgemein bekannte Verbindungen der organischen Chemie.

Die zur Durchführung des erfindungsgemäßen Verfahrens (i) als Ausgangsstoffe benötigten 5-Halogenpyrazole sind durch die Formel (VIII), allgemein definiert. In dieser Formel (VIII) stehen $R^1$, $R^2$ und Py vorzugsweise für diejenigen Reste, die bereits im Zusammenhang mit der Beschreibung der erfindungsgemäßen Stoffe der Formel (I) als bevorzugt für diese Substituenten genannt wurden, Y steht vorzugsweise für Chlor oder Brom.

Die 5-Halogen-pyrazole der Formel (VIII), sind teilweise bekannt (vgl. z.B. J. Heterocycl. Chem. 18, 9-14 (1981)). Noch nicht bekannt sind 5-Halogen-pyrazole der Formel (VIIIa)

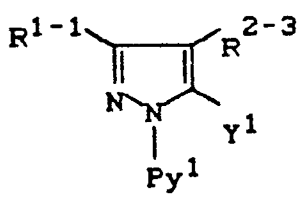

(VIIIa)

in welcher

$R^{1-1}$, $R^{2-3}$, $Y^1$ und $Py^1$ für die gleichen Reste wie die entsprechenden Reste $R^1$, $R^2$, Y und Py in der analogen Formel (VIII) stehen, wobei jedoch für den Fall, daß gleichzeitig $R^{1-1}$ für Methyl, $R^{2-3}$ für Wasserstoff und $Y^1$ für Chlor steht, $Py^1$ nicht für den 5-Nitro-2-pyridylrest steht.

Man erhält die noch nicht bekannten 5-Halogen-pyrazole der Formel (VIIIa) beispielsweise, wenn man Alkoxymethylenmalonester der Formel (XIV),

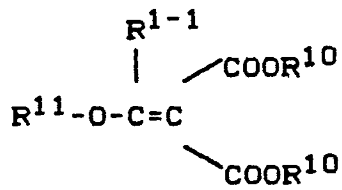

(XIV)

in welcher

$R^{1-1}$ die oben angegebene Bedeutung hat und

$R^{10}$ und $R^{11}$ unabhängig voneinander jeweils für Alkyl, insbesondere für Methyl oder Ethyl stehen,

mit Pyridylhydrazinen der Formel (IIa)

$$Py^1 - NH - NH_2$$

in welcher

$Py^1$ die oben angegebene Bedeutung hat,

zunächst in einer ersten Stufe gegebenenfalls in Gegenwart eines Verdünnungsmittels wie beispielsweise Methanol oder Ethanol bei Temperaturen zwischen +10°C und +80°C umstzt, und die so erhältlichen Pyrazolcarbonsäureester der Formel (XV)

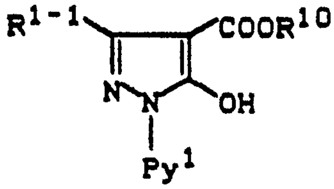

(XV)

in welcher

$R^{1-1}$, $R^{10}$ und $Py^1$ die oben angegebene Bedeutung haben,

in einer 2. Stufe gegebenenfalls in Gegenwart eines Verdünnungsmittels wie beispielsweise Methanol und gegebenenfalls in Gegenwart einer Base wie beispielsweise Natriumhydroxid bei Temperaturen zwischen +30°C und +70°C decarboxyliert zu Pyrazolinonen der Formel (XVI),

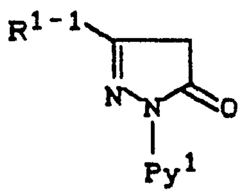

(XVI)

in welcher

$R^{1-1}$ und $Py^1$ die oben angegebene Bedeutung haben,

und diese in einer 3. Stufe mit Halogenierungsmitteln, wie beispielsweise Phosphoroxychlorid oder Phosphoroxybromid, nach üblichen, bekannten Verfahren (vgl. z.B Ber. dtsch. chem. Ges. 28, 35 (1895) oder Liebigs Ann. Chem. 373, 129 (1910)) umsetzt, und gegebenenfalls in einer 4. Stufe die so erhältlichen 5-Halogen-pyrazole der Formel (VIIIb),

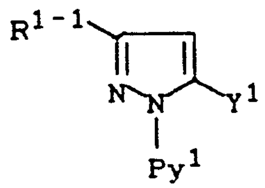

(VIIIb)

in welcher

$R^{1-1}$, $Y^1$ und $Py^1$ die oben angegebene Bedeutung haben,

in allgemein üblicher Art und Weise mit elektrophilen Agenzien der Formel (VI)

$$R^{2-1}-A^4 \qquad (VI)$$

in welcher

$R^{2-1}$ für Halogen, Nitroso, Nitro, Formyl, Alkanoyl oder Aroyl steht und

$A^4$ für eine elektronenanziehende Abgangsgruppe steht,

oder mit anderen üblichen elektrophilen Reagenzien gegebenenfalls in Gegenwart eines Verdünnungsmittels, wie beispielsweise Eisessig, und gegebenenfalls in Gegenwart eines Katalysators oder Reaktionshilfsmittels, wie beispielsweise Acetanhydrid, in Analogie zur Durchführung des erfindungsgemäßen Verfahrens (e) in 4-Stellung substituiert.

Die neuen Zwischenprodukte der Formel (VIIIa) können auch erhalten werden, indem man die erfindungsgemäßen 5-Amino-1-pyridyl-pyrazole der Formel (Ia-1)

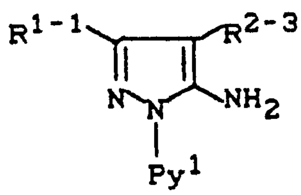

(Ia-1)

in welcher $R^{1-1}$, $R^{2-3}$ und $Py^1$ die oben angegebenen Bedeutungen haben,

in allgemein üblicher Weise in Gegenwart eines Diazotierungsmittels wie beispielsweise tert.-Butylnitrit mit Haloform-Verbindungen, wie insbesondere Bromoform, bei Temperaturen zwischen +20 und +120°C umsetzt (vgl. auch Herstellungsbeispiele).

Die bei der Umsetzung von Alkoxymethylenmalonester der Formel (XIV) mit Pyridylhydrazinen der Formel (IIa) auftretenden Zwischenprodukte der Formel (XIVa),

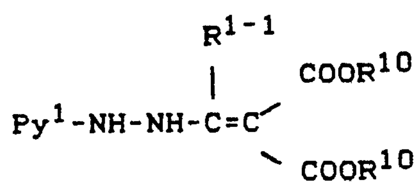

(XIVa)

in welcher

$R^{1-1}$, $R^{10}$ und $Py^1$ die oben angegebene Bedeutung haben,

können gegebenenfalls auch isoliert und in einer separaten Reaktionsstufe cyclisiert werden.

Die Cyclisierung zu den Pyrazolcarbonsäureestern der Formel (XV) und deren anschließende Decarboxylierung können gegebenenfalls in einer Reaktionsstufe als "Eintopfverfahren" durchgeführt werden (vgl. z.B. Liebigs Ann. Chem. 373, 142 (1910)).

Die Alkoxymethylenmalonester der Formel (XIV) sind allgemein bekannte Verbindungen der organischen Chemie.

Bevorzugt sind 5-Halogen-pyrazole der Formel (VIIIa), in welcher $R^{1-1}$, $R^{2-3}$ und $Py^1$ für die entsprechenden Reste $R^1$, $R^2$ und Py stehen, die bereits im Zusammenhang mit der Beschreibung der erfindungsgemäßen Stoffe der Formel (I) als bevorzugt für diese Substituenten genannt wurden, $Y^1$ steht vorzugsweise für Chlor oder Brom, wobei jedoch für den Fall, daß gleichzeitig $R^{1-1}$ für Methyl, $R^{2-3}$ für Wasserstoff und $Y^1$ für Chlor steht, $Py^1$ nicht für den 5-Nitro-2-pyridyl-Rest steht.

In der Formel (VIIIa) stehen $R^{1-1}$, $R^{2-3}$ und $Py^1$ besonders bevorzugt für die entsprechenden Reste $R^1$, $R^2$

und Py, welche bereits im Zusammenhang mit der Beschreibung der erfindungsgemäßen Stoffe der Formel (I) als besonders bevorzugt für diese Reste genannt wurden, $Y^1$ steht besonders bevorzugt für Chlor oder Brom, wobei jedoch für den Fall, daß gleichzeitig $R^{1-1}$ für Methyl, $R^{2-3}$ für Wasserstoff und $Y^1$ für Chlor steht, $Py^1$ nicht für den 5-Nitro-2-pyridyl-Rest steht.

Die zur Durchführung des erfindungsgemäßen Verfahrens (i) weiterhin als Ausgangsstoffe benötigten Amine sind durch die Formel (IX) allgemein definiert. In dieser Formel (IX) steht $R^{4-4}$ vorzugsweise für geradkettiges oder verzweigtes Alkyl mit 1 bis 6 Kohlenstoffatomen, insbesondere für Methyl, Ethyl, n- oder i-Propyl oder n-, i-, s- oder t-Butyl, $R^{3-1}$ steht vorzugsweise für Wasserstoff oder für geradkettiges oder verzweigtes Alkyl mit 1 bis 6 Kohlenstoffatomen, insbesondere für Methyl, Ethyl, n- oder i-Propyl oder n-, i-, s- oder t-Butyl.

Die Amine der Formel (IX) sind ebenfalls allgemein bekannte Verbindungen der organischen Chemie.

Die zur Durchführung des erfindungsgemäßen Verfahrens (k) als Ausgangsstoffe benötigten (Bis)Carbamate sind durch die Formel (Iw) allgemein definiert. In dieser Formel (Iw) stehen $R^1$, $R^2$ und Py vorzugsweise für diejenigen Reste, die bereits im Zusammenhang mit der Beschreibung der erfindungsgemäßen Stoffe der Formel (I) als bevorzugt für diese Substituenten genannt wurden, $R^{3-1}$ steht für einen Rest -CO-O-Ar oder für Wasserstoff, wobei Ar vorzugsweise für Phenyl steht.

Die (Bis)Carbamate der Formel (Iw) sind erfindungsgemäße Verbindungen und erhältlich mit Hilfe der erfindungsgemäßen Verfahren (d) oder (e).

Die zur Durchführung des erfindungsgemäßen Verfahrens (k) weiterhin als Ausgangsstoffe benötigten Verbindungen sind durch die Formel (X) allgemein definiert. In dieser Formel (X) steht $R^{6-1}$ vorzugsweise für jeweils geradkettiges oder verzweigtes Alkoxy, Alkylthio, Alkylamino oder Dialkylamino mit jeweils 1 bis 4 Kohlenstoffatomen in den einzelnen Alkylteilen, für jeweils einfach oder mehrfach, gleich oder verschieden substituiertes Phenoxy, Phenylthio oder Phenylamino, wobei als Phenylsubstituenten jeweils in Frage kommen: Halogen, jeweils geradkettiges oder verzweigtes Alkyl oder Alkoxy mit jeweils 1 bis 4 Kohlenstoffatomen oder Halogenalkyl mit 1 bis 4 Kohlenstoffatomen und 1 bis 9 gleichen oder verschiedenen Halogenatomen, insbesondere Methyl, Methoxy, Chlor oder Trifluormethyl. $R^{6-1}$ steht insbesondere für Methoxy, Ethoxy, Methylthio, Phenylthio oder Dimethylamino.

Die Verbindungen der Formel (X) sind allgemein bekannte Verbindungen der organischen Chemie.

Die zur Durchführung des erfindungsgemäßen Verfahrens (l) als Ausgangsstoffe benötigten 5-Sulfonamido-pyrazole sind durch die Formel (Ix) allgemein definiert. In dieser Formel (Ix) stehen $R^1$, $R^2$, $R^7$ und Py vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Sulfonamido-pyrazole der Formel (Ix) sind erfindungsgemäße Verbindungen und erhältlich mit Hilfe der erfindungsgemäßen Verfahren (d), (e) und (g).

Die zur Durchführung des erfindungsgemäßen Verfahrens (l) weiterhin als Ausgangsstoffe benötigten Salze sind durch die Formel (XI) allgemein definiert. Vorzugsweise verwendet man Alkali-, Erdalkali-, Ammonium- oder Übergangsmetallhydroxide, -oxide, -carbonate, -hydrogencarbonate oder leicht lösliche -chloride, -sulfate, -phosphate oder -nitrate, wie beispielsweise Natrium-, Kalium-, Calciumhydroxid, -carbonat oder -hydrogencarbonat, Calciumchlorid, Bariumchlorid, Kupfersulfat, Nickelchlorid oder Cobaltnitrat oder Alkylamine, wie Triethylamin, Isopropylamin, Diisopropylamin, Butylamin.

Die Salze der Formel (XI) sind allgemein bekannte Verbindungen.

Als Verdünnungsmittel zur Durchführung des Herstellungsverfahrens (a) kommen sowohl für die 1. als auch für die 2. Reaktionsstufe inerte organische Lösungsmittel in Frage. Vorzugsweise verwendet man Alkohole, wie Methanol, Ethanol, Propanol, Butanol, Ethylenglykol oder Ethylenglykolmonomethyl- oder -ethylether.

Als Reaktionshilfsmittel zur Durchführung der 1. Stufe des Herstellungsverfahrens (a) kommen organische oder anorganische Säuren in Frage. Vorzugsweise verwendet man Schwefelsäure oder Essigsäure, gegebenenfalls auch in Gegenwart einer Puffersubstanz, wie beispielsweise Natriumacetat.

Die Reaktionstemperaturen können bei der Durchführung der 1. Stufe des Herstellungsverfahrens (a) in gewissen Bereichen variiert werden. Im allgemeinen arbeitet man zwischen -30°C und +50°C, vorzugsweise zwischen -20°C und +20°C.

Als Säurebindemittel zur Durchführung der 2. Stufe des Herstellungsverfahrens (a) kommen alle üblicherweise verwendbaren anorganischen und organischen Basen in Frage. Vorzugsweise verwendet man Alkalimetallcarbonate oder Hydrogencarbonate, wie Natriumcarbonat, Kaliumcarbonat oder Kaliumhydrogencarbonat.

Das erfindungsgemäße Verfahren (a) kann auch direkt in einem Reaktionsschritt ohne Isolierung der Zwischenprodukte der Formel (IV) durchgeführt werden.

Die Reaktionstemperaturen können bei der Durchführung der 2. Stufe des Herstellungsverfahrens (a) ebenso wie bei der einstufigen Reaktionsführung in einem größeren Bereich variiert werden. Im allgemeinen arbeitet man zwischen 0°C und 200°C, vorzugsweise zwischen +50°C und +150°C.

Zur Durchführung des Herstellungsverfahrens (a) setzt man sowohl bei der einstufigen als auch bei der zweistufigen Reaktionsführung pro Mol Pyridylhydrazin der Formel (II) im allgemeinen 1,0 bis 3,0 Mol, vorzugsweise 1,0 bis 1,5 Mol an Acrylnitril-Derivat der Formel (III) und im Fall des zweistufigen Verfahrens gegebenenfalls in der 1. Stufe 1,0 bis 10,0 Mol an Reaktionshilfsmittel und gegebenenfalls in der 2. Stufe 1,0 bis 10,0 Mol an Säurebindemittel ein.

Die Aufarbeitung und Isolierung der Reaktionsprodukte der Formel (Ia) erfolgt nach üblichen Verfahren, beispielsweise durch Entfernen des organischen Verdünnungsmittels, Ausfällen des Reaktionsproduktes in Wasser, Absaugen und Trocknen des so erhaltenen Produktes.

Als Verdünnungsmittel zur Durchführung des Herstellungsverfahrens (b) kommen anorganische oder organische Lösungsmittel in Frage. Vorzugsweise verwendet man polare Lösungsmittel, insbesondere Alkohole, wie beispielsweise Methanol, Ethanol oder Propanol, oder deren Gemische mit Wasser.

Als Katalysatoren zur Durchführung des Herstellungsverfahrens (b) kommen alle üblicherweise für derartige Esterverseifungen verwendeten Katalysatoren in Frage. Vorzugsweise verwendet man Basen, wie beispielsweise Natriumhydroxid, Natriumalkoholat oder Natriumcarbonat, oder Säuren, wie beispielsweise Chlorwasserstoffsäure, Bromwasserstoffsäure oder Schwefelsäure.

Die Reaktionstemperaturen können bei der Durchführung des erfindungsgemäßen Verfahrens (b) in einem größeren Bereich variiert werden. Im allgemeinen arbeitet man bei Temperaturen zwischen 20°C und 150°C, vorzugsweise bei Temperaturen zwischen 50°C und 100°C.

Zur Durchführung des Herstellungsverfahrens (b) setzt man pro Mol an 4-Alkoxycarbonyl-5-amino-pyrazol der Formel (Ir) im allgemeinen 1,0 bis 15,0 Mol, vorzugsweise 1,0 bis 2,5 Mol an saurem oder basischem Katalysator ein und erwärmt für mehrere Stunden auf die erforderliche Reaktionstemperatur. Die Aufarbeitung, Isolierung und Reinigung der Reaktionsprodukte der Formel (Ib) erfolgt nach üblichen Verfahren.

Als Verdünnungsmittel zur Durchführung des Herstellungsverfahrens (c) kommen ebenfalls anorganische oder organische, vorzugsweise polare Lösungsmittel in Frage.

Insbesondere sind Alkohole, wie beispielsweise Methanol, Ethanol oder Propanol, oder deren Gemische mit Wasser geeignet.

Als Katalysatoren zur Durchführung des Herstellungsverfahrens (c) kommen vorzugsweise Säuren, insbesondere anorganische Mineralsäuren wie Chlorwasserstoffsäure, Bromwasserstoffsäure oder Schwefelsäure in Frage.

Die Reaktionstemperaturen können bei der Durchführung des Herstellungsverfahrens (c) in einem größeren Bereich variiert werden. Im allgemeinen arbeitet man zwischen +50°C und +200°C, vorzugsweise zwischen +70°C und +120°C.

Bei der Durchführung des erfindungsgemäßen Verfahrens (c) setzt man pro Mol an 5-Amino-1-pyridyl-pyrazol-Derivat der Formel (Ib) im allgemeinen 1,0 bis 30,0 Mol, vorzugsweise 1,0 bis 15,0 Mol an Katalysatorsäure ein und erwärmt für mehrere Stunden auf die erforderliche Temperatur. Die Aufarbeitung, Isolierung und Reinigung der Reaktionsprodukte der Formel (Ic) erfolgt nach allgemein üblichen Verfahren.

Bei Verwendung eines sauren Katalysators ist es auch möglich die erfindungsgemäßen Verfahren (b) (Esterverseifung) und (c) (Decarboxylierung) in einem Reaktionsschritt als Eintopfverfahren durchzuführen. Auch in diesem Fall erfolgt die Reaktionsdurchführung, Aufarbeitung und Isolierung der Reaktionsprodukte nach allgemein üblichen Methoden (vergl. auch Herstellungsbeispiele).

Zur Durchführung des erfindungsgemäßen Verfahrens (d) kommen als Verdünnungsmittel inerte organische Lösungsmittel infrage. Vorzugsweise verwendet man aliphatische, cyclische oder aromatische, gegebenenfalls halogenierte Kohlenwasserstoffe, wie beispielsweise Benzin, Benzol, Toluol, Xylol, Pentan, Hexan, Heptan, Cyclohexan, Petrolether, Ligroin, Methylenchlorid, Chloroform, Tetrachlorkohlenstoff, Chlorbenzol oder Dichlorbenzol, Ether, wie Diethylether, Diisopropylether, Dioxan, Tetrahydrofuran oder Ethylenglykoldiethylether oder -dimethylether, Ketone, wie Aceton, Butanon, Methylisopropylketon oder Methylisobutylketon, Ester, wie Essigsäureethylester, Nitrile, wie Acetonitril oder Propionitril, Amide, wie Dimethylformamid, Dimethylacetamid, N-Methylpyrrolidon oder Hexamethylphosphorsäuretriamid. Verwendet man Verbindungen der Formeln (V), (Va) oder (Vb) in flüssiger Form, so ist es auch möglich, diese in entsprechendem Überschuß als Verdünnungsmittel einzusetzen.

Als Säurebindemittel zur Durchführung des erfindungsgemäßen Verfahrens (d) kommen alle üblicherweise verwendbaren anorganischen und organischen Basen in Frage.

Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat, oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des Herstellungsverfahrens (d) in einem größeren Bereich variiert werden. Im allgemeinen arbeitet man zwischen -20°C und +150°C, vorzugsweise zwischen 0°C und +100°C.

Zur Durchführung des Herstellungsverfahrens (d) setzt man pro Mol 5-Amino-1-pyridyl-pyrazol der Formel (Is) im allgemeinen 1,0 bis 20,0 Mol, vorzugsweise 1,0 bis 15,0 Mol an Verbindung der Formel (V), (Va), bzw. (Vb) und gegebenenfalls 1,0 bis 3,0 Mol, vorzugsweise 1,0 bis 2,0 Mol an Säurebindemittel ein. Die Reaktionsdurchführung, Aufarbeitung und Isolierung der Reaktionsprodukte der Formel (Id) erfolgt in allgemein üblicher Art und Weise.

Als Verdünnungsmittel zur Durchführung des erfindungsgemäßen Verfahrens (e) kommen alle üblicherweise für derartige elektrophile Substitutionen verwendbaren Lösungsmittel in Frage. Vorzugsweise verwendet man die als Reagenzien in Frage kommenden Säuren oder Gemische, wie beispielsweise Schwefelsäure, Salpetersäure, Sulfurylchlorid, Phosphoroxychlorid/Dimethylformamid oder Nitriersäure, gleichzeitig als Verdünnungsmittel.

Es kommen gegebenenfalls auch inerte organische Lösungsmittel, wie beispielsweise Eisessig oder chlorierte Kohlenwasserstoffe, wie Methylenchlorid, Chloroform oder Tetrachlorkohlenstoff, als Verdünnungsmittel in Frage.

Als Katalysatoren oder Reaktionshilfsmittel zur Durchführung des Herstellungsverfahrens (e) kommen ebenfalls die für derartige Reakionen üblichen Katalysatoren in Frage; vorzugsweise verwendet man saure Katalysatoren wie beispielsweise Schwefelsäure, Eisen-III-chlorid oder andere Lewis-Säuren oder Acetanhydrid.

Die Reaktionstemperaturen können bei der Durchführung des Herstellungsverfahrens (e) in einem größeren Bereich variiert werden. Im allgemeinen arbeitet man zwischen -50°C und +200°C, vorzugsweise zwischen -20 und +150°C.

Zur Durchführung des Herstellungsverfahrens (e) setzt man pro Mol 5-Amino-1-pyridyl-pyrazol der Formel (It), im allgemeinen 1,0 bis 10,0 Mol, vorzugsweise 1,0 bis 5,0 Mol an elektrophilem Agens der Formel (VI) und gegebenenfalls 0,1 bis 10 Mol an Katalysator oder Reaktionshilfsmittel ein. Die Reaktionsführung, Aufarbeitung und Isolierung der Reaktionsprodukte der Formel (Ie) erfolgt in allgemein üblicher Art und Weise.

Als Verdünnungsmittel zur Durchführung des erfindungsgemäßen Verfahrens (f) kommen anorganische oder organische polare Lösungsmittel in Frage. Vorzugsweise verwendet man Alkohole, wie beispielsweise Methanol, Ethanol oder Propanol, oder deren Gemische mit Wasser.

Als Katalysatoren zur Durchführung des Herstellungsverfahrens (f) kommen vorzugsweise Säuren, insbesondere Chlorwasserstoffsäure oder Schwefelsäure in Frage.

Die Reaktionstemperaturen können bei der Durchführung des Herstellungsverfahrens (f) in einem größeren Bereich variiert werden. Im allgemeinen arbeitet man zwischen +20°C und +150°C, vorzugsweise zwischen +50°C und +120°C.

Zur Durchführung des Herstellungsverfahrens (f) setzt man pro Mol 5-Acylamino-1-pyridyl-pyrazol der Formel (Iu) im allgemeinen 1,0 bis 20,0 Mol, vorzugsweise 1,0 bis 10,0 Mol an Katalysatorsäure ein und erwärmt für mehrere Stunden auf die erforderliche Reaktionstemperatur. Die Aufarbeitung, Isolierung und Reinigung der Reaktionsprodukte der Formel (If) erfolgt nach üblichen Methoden.

Als Verdünnungsmittel zur Durchführung des erfindungsgemäßen Verfahrens (g) kommen polare organische Lösungsmittel oder deren Gemische mit Wasser in Frage. Vorzugsweise verwendet man Alkohole wie Methanol, Ethanol oder Propanol oder deren Gemische mit Wasser.

Als basische Reaktionsteilnehmer bei der Durchführung des erfindungsgemäßen Verfahrens (g) kommen alle üblichen anorganischen oder organischen Basen in Frage. Vorzugsweise verwendet man Amine oder Ammoniaklösungen oder Alkalimetallcarbonate bzw. -hydrogencarbonate, wie Natrium- oder Kaliumcarbonat oder Natriumhydrogencarbonat.

Die Reaktionstemperaturen können bei der Durchführung des erfindungsgemäßen Verfahrens (g) in einem größeren Bereich variiert werden. Im allgemeinen arbeitet man zwischen 0°C und 80°C, vorzugsweise zwischen 20°C und 40°C.

Zur Durchführung des erfindungsgemäßen Verfahrens (g) setzt man pro Mol 5-Bis-sulfonyl-amino-pyrazol der Formel (Iv) im allgemeinen 1,0 bis 30,0 Mol vorzugsweise 1,0 bis 15,0 Mol an Base ein.

Die Reaktionsmischung wird in einem geeigneten Verdünnungsmittel so lange gerührt (30 Minuten bis 20 Stunden) bis bei chromatographischer Kontrolle kein Ausgangsprodukt mehr nachweisbar ist. Die Aufarbeitung der Reaktionsprodukte der Formel (Ig) erfolgt nach üblichen Methoden.

Zur Durchführung des erfindungsgemäßen Verfahrens (h) kommen als Verdünnungsmittel inerte organische Lösungsmittel in Frage. Vorzugsweise verwendet man die bei Verfahren (d) genannten Verdünnungsmittel. Verwendet man die Verbindungen der Formel (VII) in flüssiger Form, so ist es auch möglich, diese in entsprechendem Überschuß als Verdünnungsmittel einzusetzen.

Als Reaktionshilfsmittel zur Durchführung des erfindungsgemäßen Verfahrens (h) kommen tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU), in Frage.

Die Reaktionstemperaturen können bei der Durchführung des Verfahrens (h) in einem größeren Bereich variiert werden. Im allgemeinen arbeitet man zwischen -20° C und +150° C, vorzugsweise zwischen 0° C und +100° C.

Zur Durchführung des Herstellungsverfahrens (h) setzt man pro Mol 5-Amino-1-pyridyl-pyrazol der Formel (Is) im allgemeinen 1,0 bis 20,0 Mol, vorzugsweise 1,0 bis 15,0 Mol an Verbindung der Formel (VII)und gegebenenfalls 1,0 bis 3,0 Mol, vorzugsweise 1,0 bis 2,0 Mol an Reaktionshilfsmittel ein. Die Reaktionsdurchführung, Aufarbeitung und Isolierung der Reaktionsprodukte der Formel (Ih) erfolgt in allgemein üblicher Art und Weise.

Als Verdünnungsmittel zur Durchführung des erfindungsgemäßen Verfahrens (i) kommen inerte organische Lösungsmittel in Frage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren (i) kann gegebenenfalls in Gegenwart eines geeigneten Säurebindemittels durchgeführt werden.
Als solche kommen alle üblichen anorganischen oder organischen Basen infrage. Hierzu gehören beispielsweise Alkalimetallhydroxide, wie Natriumhydroxid oder Kaliumhydroxid, Alkalimetallcarbonate, wie Natriumcarbonat,

Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Es ist jedoch auch möglich, einen entsprechenden Überschuß an dem als Reaktionspartner eingesetzten Amin der Formel (IX) gleichzeitig als Säurebindemittel zu verwenden.

Die Reaktionstemperaturen können bei der Durchführung des erfindungsgemäßen Verfahrens (i) in einem größeren Bereich variiert werden. Im allgemeinen arbeitet man bei Temperaturen zwischen -20° C und +200° C, vorzugsweise bei Temperaturen zwischen 0° C und +150° C.

Zur Durchführung des erfindungsgemäßen Verfahrens (i) setzt man pro Mol an 5-Halogen-pyrazol der Formel (VIII) im allgemeinen 1,0 bis 10,0 Mol, vorzugsweise 1,0 bis 5,0 Mol an Amin der Formel (IX) ein. Die Reaktionsdurchführung, Aufarbeitung und Isolierung der Reaktionsprodukte der Formel (Ii) erfolgt nach allgemein üblichen Verfahren.

Als Verdünnungsmittel zur Durchführung des erfindungsgemäßen Verfahrens (k) kommen inerte organische Lösungsmittelin Frage. Vorzugsweise verwendet man aliphatische, oder aromatische, gegebenenfalls halogenierte Kohlenwasserstoffe, wie Benzin, Benzol, Toluol, Xylol,

Pentan, Hexan, Heptan, Cyclohexan, Petrolether, Ligroin, Methylenchlorid, Ethylenchlorid, Chloroform, Tetrachlorkohlenstoff,Chlorbenzol oder Dichlorbenzol, Ether, wie Diethylether oder Diisopropylether, Ethylenglykoldimethylether, Tetrahydrofuran oder Dioxan, Ketone, wie Aceton oder Butanon, Methylisopropylketon oder Methylisobutylketon, Ester, wie Essigsäureethylester, Nitrile, wie Acetonitril oder Propionitril, Amide, wie Dimethylformamid, Dimethylacetamid, N-Methylpyrrolidon oder Hexamethylphosphorsäuretriamid, oder Alkohole wie Methanol, Ethanol oder Isopropanol.

Es ist jedoch auch möglich, die als Reaktionskomponenten verwendeten Verbindungen der Formel (X) in entsprechendem Überschuß gleichzeitig als Verdünnungsmittel einzusetzen.

Das erfindungsgemäße Verfahren (k) kann gegebenenfalls in Gegenwart eines basischen Katalysators durchgeführt werden. Als solche kommen alle üblichen anorganischen oder organischen Basen infrage. Vorzugsweise verwendet man die bei Verfahren (i) genannten Basen.

Die Reaktionstemperaturen können bei dem erfindungsgemäßen Verfahren (k) ebenfalls in einem größeren Bereich variiert werden. Im allgemeinen arbeitet man zwischen 0°C und +200°C, vorzugsweise zwischen +20°C und +150°C.

Zur Durchführung des erfindungsgemäßen Verfahrens (k) setzt man pro Mol (Bis)Carbamat der Formel (Iw) im allge-

meinen 1 bis 20 Mol, vorzugsweise 1 bis 10 Mol der Verbindung der Formel (X) ein und erwärmt für mehrere Stunden auf die erforderliche Temperatur. Die Aufarbeitung und Isolierung der Reaktionsprodukte der Formel (Ik) erfolgt nach üblichen Verfahren.

Als Verdünnungsmittel zur Durchführung des erfindungsgemäßen Verfahrens (l) kommen polare organische Lösungsmittel, Wasser oder wäßrige Gemische, in Frage. Vorzugsweise verwendet man Alkohole, wie beispielsweise Methanol, Ethanol oder Propanol, deren Gemische mit Wasser oder reines Wasser.

Die Reaktionstemperaturen können bei der Durchführung des Herstellungsverfahrens (l) ebenfalls in einem größeren Bereich variiert werden. Im allgemeinen arbeitet man zwischen 0°C und +80°C, vorzugsweise zwischen +20°C und +40°C.

Zur Durchführung des erfindungsgemäßen Verfahrens (l) setzt man pro Mol 5-Sulfonamido-pyrazol der Formel (Ix) im allgemeinen 1,0 bis 10 Mol, vorzugsweise 1,0 bis 5,0 Mol an Salz der Formel (XI) oder an Amin ein.

Zur Herstellung der Natrium-, Kalium- oder Ammoniumsalze setzt man eine Verbindung der Formel (Ix) in wäßriger Lösung oder einem organischen Lösungsmittel, wie Aceton, Methanol, Ethanol oder Dimethylformamid, mit Natrium-, Kalium- oder Ammoniumhydroxid oder einem Amin um und isoliert die Salze durch Abfiltrieren oder durch

Eindampfen der Lösung und reinigt sie gegebenenfalls durch Umkristallisieren.

Die Calcium-, Barium-, Magnesium-, Mangan-, Kupfer-, Nickel-, Zinn-, Eisen- oder Cobaltsalze werden hergestellt aus den Natriumsalzen durch Behandeln mit einem entsprechenden anorganischen Metallsalz, z.B. Calciumchlorid, Bariumchlorid, Kupfersulfat, Nickelchlorid oder Cobaltnitrit. Die Calciumsalze können auch hergestellt werden durch Behandeln einer Verbindung der Formel (Ix) mit Calciumhydroxid.
Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala,

Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotina, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-,

Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung mono- und dikotyler Unkräuter in monokotylen und dikotylen Kulturen wie beispielsweise Weizen oder Baumwolle einsetzen.

Auch die Zwischenprodukte der Formel (VIII) besitzen eine hohe herbizide Wirksamkeit.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polaren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmitteln können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablauge und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage.
Auch Mischungen mit N,N-Dimethyl-N'-(3-trifluormethylphenyl)-harnstoff; N,N-Dimethyl-N'-(3-chlor-4-methylphenyl)-harnstoff; N,N-Dimethyl-N'-(4-isopropylphenyl)-harnstoff; 2,4-Dichlorphenoxyessigsäure; 2,4-Dichlorphenoxypropionsäure; (2-Methyl-4-chlorphenoxy)-essigsäure; (4-Chlor-2-methyl-phenoxy)-propionsäure; 2-[4-(3,5-Dichlorpyrid-2-yloxy)-phenoxy]-propionsäure-(2-benzyloxy-ethylester), -(trimethylsilylmethylester) oder -(2,2-diethoxyethylester);Methyl-5-(2,4-dichlorphenoxy)-2-nitrobenzoat; 3,5-Diiod-4-hydroxybenzonitril; 2-Chlor-N-{[(4-methoxy-6-methyl-1,3,5-triazin-2-yl)-amino]-carbonyl}-benzolsulfonamid, 4-Ethylamino-2-t-butylamino-6-methylthio-s-triazin; N-Methyl-2-(benzthiazol-2-yloxy)-acetamid; N-(1-Ethylpropyl)-3,4-dimethyl-2,6-dinitroanilin; Chloressigsäure-N-(methoxymethyl)-2,6-diethylanilid; 2-Ethyl-6-methyl-N-(1-methyl-2-methoxyethyl)-chloracetanilid; 2,6-Dinitro-4-trifluormethyl-N,N-dipropylanilin; 2-{4-[[3-Chlor-5-(trifluormethyl)-2-pyridinyl]-oxy]-phenoxy}-propansäureethylester oder N,N-Diisopropyl-(2,3,3-trichlorallyl)-thiocarbamat sowie weiteren Triazinonen, sind möglich. Einige Mischungen zeigen überraschenderweise auch synergistische Wirkung.

Auch Mischungen mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln sind möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

0207285

Herstellungsbeispiele:

Beispiel 1:

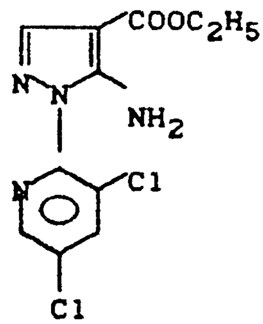

(Verfahren a)

16,9 g (0,1 Mol) Ethoxymethylencyanessigsäureethylester und 17,8g (0,1 Mol) 3,5-Dichlor-pyrid-2-ylhydrazin in 150 ml Ethoxyethanol werden 5 Stunden bei 80°C und danach weitere 2 Stunden bei 120°C gerührt. Zur Aufarbeitung entfernt man das Lösungsmittel im Vakuum. Man erhält 29,6 g (98 % der Theorie) an 5-Amino-1-(3,5-dichlor-pyrid- 2-yl)-4-ethoxycarbonyl-pyrazol vom Schmelzpunkt 98-101°C.

Beispiel 2:

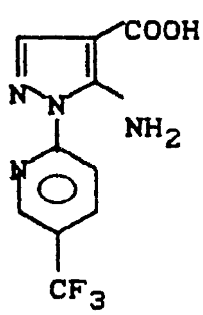

(Verfahren b)

18 g (0,06 Mol) 5-Amino-1-(5-trifluormethyl-pyrid-2-yl)-4-ethoxycarbonyl-pyrazol in 100 ml 50-%iger wässriger Ethanollösung werden mit 10 ml 45-%iger wässriger Natriumhydroxidlösung versetzt und 4 Stunden bei 80°C gerührt. Zur Aufarbeitung entfernt man das Lösungsmittel im Vakuum, nimmt den Rückstand in 50 ml Wasser auf und rührt die Mischung in eine Lösung aus 20 ml konzentrierter Salzsäure und 50 ml Wasser ein. Der Niederschlag wird abgesaugt, mit wenig verdünnter Salzsäure gewaschen und im Vakuum bei 50°C getrocknet. Man erhält 15,4g (94,4 % der Theorie) an 5-Amino-1-(5-trifluormethyl-pyrid-2-yl)-pyrazol-4-carbonsäure vom Schmelzpunkt 196°C (Zers.).

Beispiel 3:

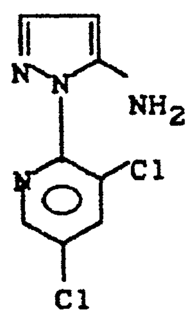

(Verfahren c)

16,5g (0,06 Mol) 5-Amino-1-(3,5-dichlorpyrid-2-yl)-pyrazol-4-carbonsäure werden in einem Gemisch aus 150 ml Wasser, 75 ml konzentrierter Salzsäure und 20 ml Isopropanol langsam auf 80°C erhitzt und so lange bei dieser Temperatur gerührt, bis die Gasentwicklung beendet ist. Die entstandene klare Lösung wird zur Trockne eingedampft, der Rückstand in Dichlormethan suspendiert,

die Suspension mit wässriger Natriumcarbonatlösung neutralisiert, die organische Phase abgetrennt und die wässrige Phase noch zweimal mit Dichlormethan extrahiert. Die vereinigten organischen Phasen werden über Natriumsulfat getrocknet und im Vakuum vom Lösungsmittel befreit. Man erhält 12,7g (92 % der Theorie) an 5-Amino-1-(3,5-dichlorpyrid-2-yl)-pyrazol als Öl. $^1$H-NMR ($CDCl_3$/TMS als innerer Standard): δ=4.55; 5.55; 7,43; 7.88; 8.30 ppm.

<u>Beispiel 4:</u>

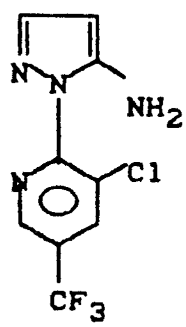

(Verfahren (b) und (c) als "Eintopfreaktion")

10,5g (0,03 Mol) 5-Amino-1-(3-chlor-5-trifluormethyl-pyrid-2-yl)-4-ethoxycarbonyl-pyrazol in 100 ml wässriger 48-%iger Bromwasserstoffsäure werden langsam auf 80°C erwärmt. Die heftige Gasentwicklung wird durch Zugabe von 5 ml Isopropanol gedämpft und die Reaktionsmischung langsam weiter auf 115°C bis 120°C erhitzt. Nach ca. 2 Stunden ist die Gasentwicklung beendet. Man rührt weitere 3 Stunden bei 115°C bis 120°C, entfernt die Bromwasserstoffsäure im Vakuum, nimmt den Rückstand in ca. 200 ml Dichlormethan auf und neutralisiert mit wässriger Natriumhydrogencarbonatlösung. Die organische Phase wird

abgetrennt und die wässrige Phase noch zweimal mit Dichlormethan extrahiert; die vereinigten organischen Phasen werden über Magnesiumsulfat getrocknet und im Vakuum vom Lösungsmittel befreit.
Man erhält 4,7 g (60 % der Theorie) an 5-Amino-1-(3-chlor-5-trifluormethyl-pyrid-2-yl)-pyrazol als Öl.
$^1$H-NMR ($CDCl_3$/TMS): $\delta$= 4.1-5.0; 5.6; 7.5; 8.1-8.6 ppm.

Beispiel 5:

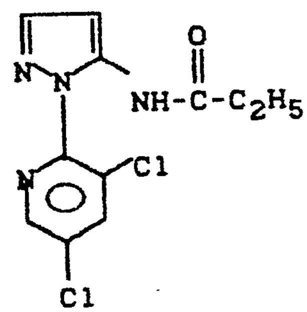

(Verfahren d)
11 g (0.048 Mol) 5-Amino-1-(3,5-dichlorpyrid-2-yl)--pyrazol in 80 ml Dichlormethan werden bei Raumtemperatur unter Rühren nacheinander tropfenweise mit 4,3 ml (0,053 Mol) Pyridin und 3,6 ml (0,051 Mol) Propionylchlorid versetzt. Nach beendeter Zugabe rührt man weitere 5 Stunden, verdünnt mit 70 ml Dichlormethan, wäscht nacheinander mit verdünnter Salzsäure, gesättigter Natriumhydrogencarbonatlösung und Natriumchloridlösung, trocknet über Magnesiumsulfat und entfernt das Lösungsmittel im Vakuum. Man erhält 12,5g (91,4 % der Theorie) an 5-Propionamido-1-(3,5-Dichlor- pyrid-2-yl)-pyrazol vom Schmelzpunkt116-124°C.

Beispiel 6:

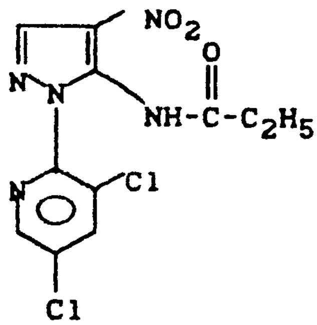

(Verfahren e)

9,0 g (0,032 Mol) 5-Propionamido-1-(3,5-dichlor-pyrid-2-yl)-pyrazol und 3,2 ml (0,035 Mol) Acetanhydrid in 35 ml Eisessig werden bei 10°C mit 1,5 ml (0,033 Mol) 98-%iger Salpetersäure versetzt und 6 Stunden bei Raumtemperatur gerührt. Zur Aufarbeitung wird die Mischung im Vakuum eingeengt, der Rückstand in 100 ml Dichlormethan aufgenommen, mit Natriumhydrogencarbonatlösung neutralisiert, mit Kochsalzlösung gewaschen, über Magnesiumsulfat getrocknet und im Vakuum vom Lösungsmittel befreit. Man erhält 9,3g (89 % der Theorie) an 5-Propionamido-1-(3,5-dichlorpyrid-2-yl)-4-nitro-pyrazol vom Schmelzpunkt 53°C-56°C.

Beispiel 7:

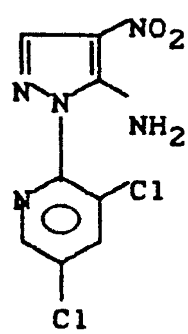

(Verfahren f)
5 g (0,015 Mol) 5-Propionamido-1-(3,5-dichlorpyrid-2-yl)-4-nitro-pyrazol und 10 ml konzentrierte Salzsäure in 15 ml Ethanol werden 4 Stunden unter Rückfluß erhitzt, das Lösungsmittel im Vakuum abdestilliert, der Rückstand in 400 ml Dichlormethan aufgenommen, mit Natriumhydrogencarbonatlösung neutralisiert, mit Kochsalzlösung gewaschen, über Natriumsulfat getrocknet und im Vakuum vom Lösungsmittel befreit. Man erhält 3,8 g (92 % der Theorie) an 5-Amino-1-(3,5-dichlorpyrid-2-yl)-4- nitro-pyrazol vom Schmelzpunkt 194°C.

In entsprechender Weise und gemäß den allgemeinen Angaben zur Herstellung erhält man die folgenden 5-Amino-1-pyridyl-pyrazole der allgemeinen Formel (I):

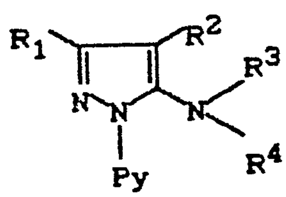

(I)

**Tabelle 2:**

| Bsp. Nr. | $R^1$ | $R^2$ | $R^3$ | $R^4$ | Py | physikal. Konstante |
|---|---|---|---|---|---|---|
| 8 | H | $C_2H_5O-CO-$ | H | H | 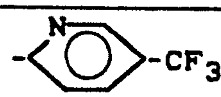 | Fp: 130-132°C |
| 9 | H | $C_2H_5O-CO-$ | H | H | 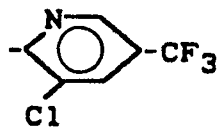 | Fp: 104-110°C |
| 10 | H | HOOC | H | H | 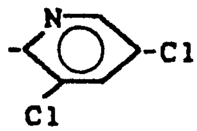 | Fp: 187°C (Zers.) |
| 11 | H | H | H | H | 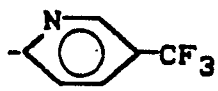 | Fp: 77-79°C |
| 12 | H | H | $-CO-C_2H_5$ | H | 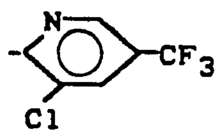 | Fp: 80-82°C |
| 13 | H | H | $-CO-C_2H_5$ | H | 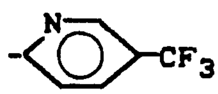 | Fp: 110-114°C |
| 14 | H | $NO_2$ | $-CO-C_2H_5$ | H | 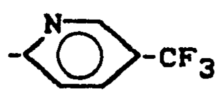 | Fp: 128-130°C |
| 15 | H | $NO_2$ | $-CO-C_2H_5$ | H | 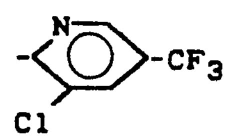 | Fp: 114-118°C |

Tabelle 2 (Fortsetzung)

| Bsp. Nr. | $R^1$ | $R^2$ | $R^3$ | $R^4$ | Py | physikal. Konstante |
|---|---|---|---|---|---|---|
| 16 | H | $NO_2$ | H | H | 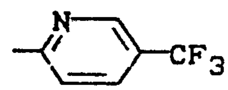 | Fp: 114° C |
| 17 | H | $NO_2$ | H | H | 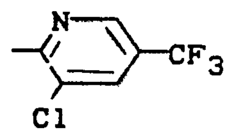 | Fp: 79-84° C |
| 18 | H | H | $-CO-CHCl_2$ | H | 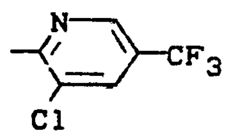 | Fp: 79-84° C |
| 19 | H | $NO_2$ | $-CO-CHCl_2$ | H | 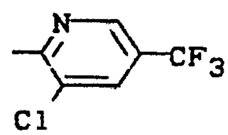 | Fp: 127-132° C |
| 20 | H | H | $-CO-CHCl_2$ | H | 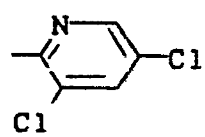 | Fp: 106° C |
| 21 | H | $NO_2$ | $-CO-CHCl_2$ | H | 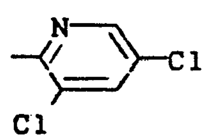 | Fp: 112° C |

0207285

Beispiel 22

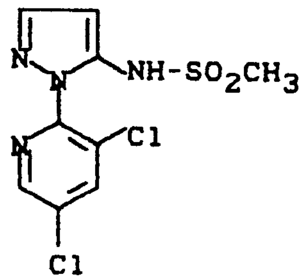

(Verfahren d/Variante β)

15 g (0,066 Mol) 5-Amino-1-(3,5-dichlorpyrid-2-yl)-pyrazol werden in 65 ml wasserfreiem Pyridin gelöst und bei 0°C - 5°C innerhalb von 20 Minuten mit 9,2 ml (0,115 Mol) Methansulfonsäurechlorid versetzt. Es wird 1 Stunde nachgerührt. Danach wird die Reaktionsmischung auf eine Mischung aus Methylenchlorid und Eiswasser ausgetragen. Die organische Phase wird abgetrennt, mit verdünnter Salzsäure gewaschen und im Vakuum vom Lösungsmittel befreit. Der Rückstand wird in 200 ml Ethanol gelöst, mit 150 ml konzentriertem wäßrigen Ammoniak versetzt und 18 Stunden bei Raumtemperatur gerührt. Das Ethanol wird im Vakuum abdestilliert, der Rückstand in Dichlormethan aufgenommen, mit verdünnter Salzsäure gewaschen und über Magnesiumsulfat getrocknet und im Vakuum vom Lösungsmittel befreit.
Man erhält 17,5 g (87 % der Theorie) an 1-(3,5-Dichlorpyrid-2-yl)-5-methansulfonamido-pyrazol vom Schmelzpunkt 52-54°C.

Beispiel 23

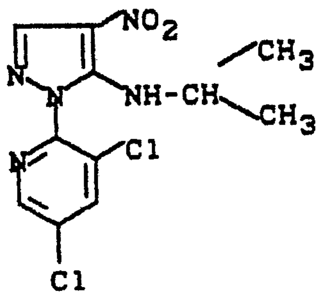

(Verfahren i)

Ein Gemisch aus 5,1 g (0,015 Mol) 1-(3,5-Dichlorpyrid-2-yl)-4-nitro-5-brom-pyrazol und 13,4 g (0,225 Mol) Isopropylamin in 100 ml Methylenchlorid wird 20 Stunden bei Raumtemperatur gerührt. Die Reaktionsmischung wird im Vakuum eingeengt, der Rückstand in 50 ml Methylenchlorid aufgenommen und zweimal mit 50 ml Wasser gewaschen; die organische Phase wird über Natriumsulfat getrocknet und das Lösungsmittel im Vakuum abdestilliert. Der Rückstand wird durch Anreiben mit Petrolether zur Kristallisation gebracht, abgesaugt und getrocknet. Man erhält 4,0 g (84,4 % der Theorie) an 1-(3,5-(Dichlor-pyrid-2-yl)-4-nitro-5-isopropylamino-pyrazol vom Schmelzpunkt 123° C.

Herstellung des Ausgangsprocuktes

Beispiel VIIIa-1

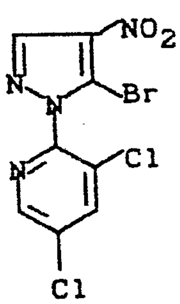

Zu 54,8 g (0,2 Mol) 1-(3,5-Dichlor-pyrid-2-yl)-4-nitro-5-amino-pyrazol in 250 ml (2,26 Mol) Bromoform werden innerhalb von 30 Minuten 72 ml (0,96 Mol) tert.-Butylnitrit getropft, während des Zutropfens steigt die Temperatur des Reaktionsgemisches auf 80° C an. Es wird weitere 2 Stunden bei 80° C gerührt. Nach dem Abkühlen wird im Vakuum eingeengt. Der Rückstand wird durch Anreiben mit Petrolether kristallisiert, abgesaugt und getrocknet.

Man erhält 64 g (94,7 % der Theorie) an 1-(3,5-Dichlor-pyrid-2-yl)-4-nitro-5-brompyrazol vom Schmelzpunkt 122° C.

Beispiel 24

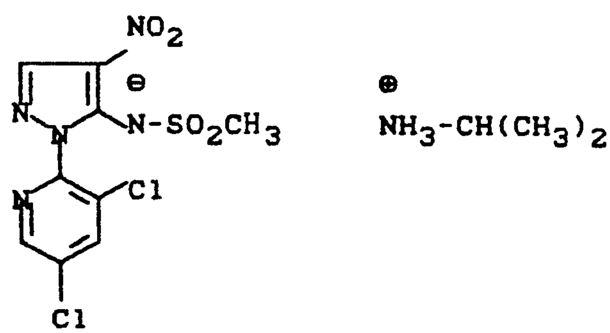

(Verfahren 1)

2 g (5,7 mMol) 1-(3,5-Dichlorpyrid-2-yl)-5-methansulfonamido-4-nitro-pyrazol werden in 30 ml Ethanol suspendiert und mit 0,75 ml (8,5 mMol) wasserfreiem Isopropylamin versetzt. Es entsteht eine klare Lösung, aus der im Vakuum das Lösungsmittel abdestilliert wird.

Man erhält 2,3 g (98,5 % der Theorie) an 1-(3,5-Dichlorpyrid-2-yl)-5-methansulfonamido-4-nitro-pyrazol Isopropylammoniumsalz vom Schmelzpunkt 99-109° C.

Tabelle 2 (Fortsetzung)

| Bsp. Nr. | $R^1$ | $R^2$ | $R^3$ | $R^4$ | Py | physikal. Konstante |
|---|---|---|---|---|---|---|
| 25 | H | $NO_2$ | H | $-(CH_2)_3CH_3$ | 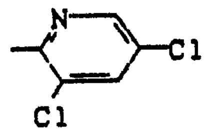 | Fp: 77° C |
| 26 | H | $NO_2$ | $-SO_2-CH_3$ | H | 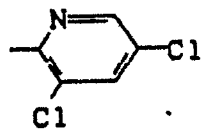 | Fp: 85-123° C |
| 27 | H | $NO_2$ | H | $-(CH_2)_2CH_3$ | 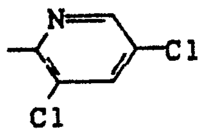 | Fp: 87° C |

## Anwendungsbeispiele

In den folgenden Anwendungsbeispielen wurde die nachstehend aufgeführte Verbindung als Vergleichssubstanz eingesetzt:

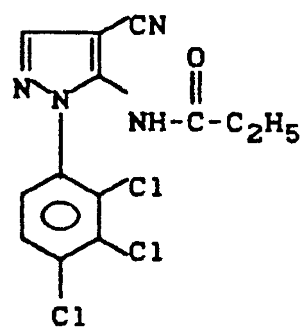

(A)

4-Cyano-5-propionamido-1-(2,3,4-trichlorphenyl)-pyrazol
(bekannt aus DE-OS 32 26 513)

**Beispiel A**

Pre-emergence-Test / Gewächshaus

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen boniert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Nutzpflanzenselektivität gegenüber dem Stand der Technik zeigt in diesem Test z.B. die Verbindung gemäß den Herstellungsbeispielen: 7, 19 und 27.

Beispiel B

Post-emergence-Test / Gewächshaus

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandeltenKontrolle. Es bedeuten:

0 % = keine Wirkung (wie unbehandelte Kontrolle)
100 % = totale Vernichtung

Eine deutliche Überlegenheit in der Wirksamkeit sowie in der Nutzpflanzenselektivität gegenüber dem Stand der Technik zeigt in diesem Test z.B. die Verbindung gemäß dem Herstellungsbeispiel: 7.

Patentansprüche

1. 5-Amino-1-pyridyl-pyrazole der Formel (I),

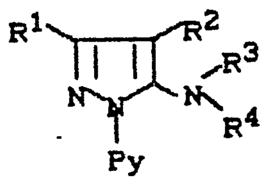

(I)

in welcher

$R^1$ für Wasserstoffoder für Alkyl mit 1 bis 12 Kohlenstoffatomen steht,

$R^2$ für Wasserstoff, Nitro, Nitroso, Halogen oder für einen Rest $-\underset{\underset{O}{\|}}{C}-R^5$ steht, wobei

$R^5$ für Wasserstoff, Hydroxy, Alkyl, Alkenyl, Alkinyl, Halogenalkyl, Alkoxyalkyl, Alkylthioalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht,

$R^3$ für Wasserstoff, für einen Rest $-\overset{\overset{X}{\|}}{C}-R^6$ oder für einen Rest $-S(O)_n-R^7$ steht,

$R^4$ für Wasserstoff, für Alkyl, für einen Rest $-\overset{X}{\overset{\|}{C}}-R^6$ oder für einen Rest $-S(O)_n-R^7$ steht, und für den Fall, daß $R^3$ für einen $-SO_2-R^7$-Rest steht auch für ein salzartig gebundenes anorganisches oder organisches Kation steht,

$R^6$ für Wasserstoff, Alkyl, Alkenyl, Alkinyl, Halogenalkyl, Alkoxyalkyl, Alkylthioalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht,

X für Sauerstoff oder Schwefel steht,

n für eine Zahl 0, 1 oder 2 steht,

$R^7$ für Alkyl, Halogenalkyl oder für gegebenenfalls substituiertes Aryl steht und

Py für substituiertes C-verknüpftes Pyridyl steht,

2. 5-Amino-1-pyridylpyrazole der Formel (I) gemäß Anspruch 1, in welcher

$R^1$ für Wasserstoff oder für geradkettiges oder verzweigtes Alkyl mit 1 bis 8 Kohlenstoffatomen steht,

$R^2$ für Wasserstoff, Nitro, Nitroso, Fluor, Chlor, Brom, Iod oder für einen Rest $-\underset{\underset{O}{\|}}{C}-R^5$ steht, wobei

$R^5$ für Wasserstoff, Hydroxy, geradkettiges oder verzweigtes Alkyl mit 1 bis 12 Kohlenstoffatomen, für jeweils geradkettiges oder verzweigtes Alkenyl, Alkinyl, Alkoxyalkyl, Alkylthioalkyl, Alkoxy, Alkylthio, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht, außerdem für gegebenenfalls einfach oder mehrfach, gleich oder verschieden durch Halogen, $C_1$-$C_4$-Alkyl oder $C_1$-$C_4$-Halogenalkyl substituiertes Cycloalkyl mit 3 bis 7 Kohlenstoffatomen, sowie für jeweils gegebenenfalls einfach oder mehrfach, gleich oder verschieden substituiertes Phenyl, Phenoxy, Phenylthio oder Phenylamino steht, wobei als Phenylsubstituenten jeweils in

in Frage kommen: Halogen, jeweils geradkettiges oder verzweigtes Alkyl, Alkoxy mit jeweils 1 bis 4 Kohlenstoffatomen und Halogenalkyl mit 1 bis 4 Kohlenstoffatomen und 1 bis 9 gleichen oder verschiedenen Halogenatomen,

$R^3$ für Wasserstoff, für einen Rest $-\underset{\underset{X}{\|}}{C}-R^6$ oder für oder für einen Rest $-S(O)_n-R^7$ steht,

$R^4$ für Wasserstoff, für einen Rest $-\underset{\underset{X}{\|}}{C}-R^6$ oder für einen Rest $-S(O)_n-R^7$ steht, für geradkettiges oder verzweigtes Alkyl mit 1 bis 6 Kohlenstoffatomen steht, oder für den Fall, daß $R^3$ für einen Rest $-SO_2-R^7$ steht auch für ein salzartig gebundenes Äquivalent eines Alkali- oder Erdalkali- oder Übergangsmetallkations oder für ein gegebenenfalls durch $C_1$-$C_4$-Alkyl oder Phenyl substituiertes Ammoniumion steht, wobei

$R^6$ für Wasserstoff, für geradkettiges oder verzweigtes Alkyl mit 1 bis 12 Kohlenstoffatomen, für jeweils geradkettiges oder verzweigtes Alkenyl oder Alkinyl mit jeweils 2 bis 4 Kohlenstoffatomen, für jeweils geradkettiges oder verzweigtes Alkoxyalkyl, Alkylthioalkyl, Alkoxy, Alkylthio, Alkylamino, Dialkylamino oder Halogenalkyl mit jeweils 1 bis 4 Kohlenstoffatomen

0207285

in den einzelnen Alkylteilen und im Fall des Halogenalkyl mit 1 bis 9 gleichen oder verschiedenen Halogenatomen steht, außerdem für gegebenenfalls einfach oder mehrfach, gleich oder verschieden durch Halogen, $C_1$-$C_4$-Alkyl oder $C_1$-$C_4$-Halogenalkyl substituiertes Cycloalkyl mit 3 bis 7 Kohlenstoffatomen, sowie für jeweils gegebenenfalls einfach oder mehrfach, gleich oder verschieden substituiertes Phenyl, Phenoxy, Phenylthio oder Phenylamino steht, wobei als Phenylsubstituenten jeweils in Frage kommen: Halogen, jeweils geradkettiges oder verzweigtes Alkyl, Alkoxy mit jeweils 1 bis 4 Kohlenstoffatomen und Halogenalkyl mit 1 bis 4 Kohlenstoffatomen und 1 bis 9 gleichen oder verschiedenen Halogenatomen,

$R^7$ für jeweils geradkettiges oder verzweigtes Alkyl oder Halogenalkyl mit jeweils 1 bis 4 Kohlenstoffatomen und gegegebenenfalls 1 bis 9 gleichen oder verschiedenen Halogenatomen oder für gegebenenfalls einfach oder mehrfach, gleich oder verschieden substituiertes Phenyl steht, wobei als Phenylsubstituenten in Frage kommen: Halogen, jeweils geradkettiges oder verzweigtes Alkyl, Alkoxy mit jeweils 1 bis 4 Kohlenstoffatomen und Halogenalkyl mit 1 bis 4 Kohlenstoffatomen und 1 bis 9 gleichen oder verschiedenen Halogenatomen,

X für Sauerstoff oder Schwefel steht,

n für eine Zahl 0, 1 oder 2 steht und

Py für jeweils einfach oder mehrfach, gleich oder verschieden substituiertes 2-Pyridyl, 3-Pyridyl oder 4-Pyridyl steht, wobei als Substituenten in Frage kommen: Cyano, Nitro, Halogen, jeweils geradkettiges oder verzweigtes Alkyl, Alkoxy oder Alkoxycarbonyl mit jeweils 1 bis 4 Kohlenstoffatomen im Alkylteil, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_m-R^9$, wobei

$R^9$ für Amino, für jeweils geradkettiges oder verzweigtes Alkyl, Alkylamino oder Dialkylamino mit 1 bis 4 Kohlenstoffatomen in den einzelnen Alkylteilen oder für Halogenalkyl mit 1 bis 4 Kohlenstoffatomen und mit 1 bis 9 gleichen oder verschiedenen Halogenatomen steht und

m für eine Zahl 0, 1 oder 2 steht.

3. 5-Amino-1-pyridyl-pyrazole der Formel (I) gemäß Anspruch 1, in welcher

$R^1$ für Wasserstoff, Methyl, Ethyl, n- oder i-Propyl, n-, i-, s- oder t-Butyl steht,

$R^2$ für Wasserstoff, Nitro, Nitroso, Fluor, Chlor, Brom, Iod oder für einen Rest $-\underset{\underset{O}{\|}}{C}-R^5$ steht, wobei

$R^5$ für Wasserstoff, Hydroxy, Methyl, Ethyl, n- oder i-Propyl, n-, i-, s- oder t-Butyl, n- oder i-Pentyl, Vinyl, Allyl, Propargyl, Butenyl, Methoxymethyl, Ethoxymethyl, Methoxyethyl, Ethoxyethyl, Methylthiomethyl, Methoxy, Ethoxy, Methylthio, Ethylthio, Methylamino, Ethylamino, Dimethylamino, Diethylamino, Trifluormethyl, Trichlorethyl, Dichlorfluorethyl, Difluorchlorethyl, Chlormethyl, Iodmethyl, Brommethyl, Dichlormethyl, 1-Chlorethyl, 2-Chlorethyl, 2-Bromethyl, 2-Chlorpropyl, Heptafluor-n-propyl, für jeweils gegebenenfalls ein- bis vierfach, gleich oder verschieden durch Fluor, Chlor, Brom, Methyl oder Trifluormethyl substituiertes Cyclopropyl, Cyclopentyl oder Cyclohexyl, für jeweils gegebenenfalls ein-bis dreifach, gleich oder verschieden durch Methyl, Methoxy, Chlor oder Trifluormethyl substituiertes Phenyl, Phenoxy, Phenylthio oder Phenylamino steht,

$R^3$ für Wasserstoff, für einen Rest $-\underset{\underset{X}{\|}}{C}-R^6$ oder für einen Rest $-S(O)_n-R^7$ steht,

$R^4$ für Wasserstoff, für einen Rest $-\overset{\overset{X}{\|}}{C}-R^6$ oder für einen Rest $-S(O)_n-R^7$ steht, für Methyl, Ethyl, n- oder i-Propyl, n-, i-, s- oder t-Butyl steht oder für den Fall, daß $R^3$ für einen Rest $-SO_2-R^7$ steht, auch für ein salzartig gebundenes Äquivalent eines Natrium-, Kalium-, Magnesium-, Calcium-, Barium-, Kupfer-, Zink-, Mangan-, Zinn-, Eisen-, Cobalt- oder Nickelions steht, oder für ein gegebenenfalls ein- bis dreifach, gleich oder verschieden durch Methyl, Ethyl, n- oder i-Propyl, n-, i-, s- oder t-Butyl oder Phenyl substituiertes Ammoniumion steht, wobei

$R^6$ für Wasserstoff, Methyl, Ethyl, n- oder i-Propyl, n-, i-, s- oder t-Butyl, n- oder i-Pentyl n- oder i-Hexyl, n- oder i-Heptyl, Vinyl, Allyl, Propargyl, Butenyl, Methoxymethyl, Ethoxymethyl, Methoxyethyl, Ethoxyethyl, Methylthiomethyl, Methoxy, Ethoxy, Methylthio, Ethylthio, Methylamino, Ethylamino, Dimethylamino, Diethylamino, Trifluormethyl, Trichlorethyl, Dichlorfluorethyl, Difluorchlorethyl, Chlormethyl, Iodmethyl, Brommethyl, Dichlormethyl, 1-Chlorethyl, 2-Chlorethyl, 2-Bromethyl, 3-Chlorpropyl, Heptafluor-n-propyl, für

jeweils gegebenenfalls ein- bis vierfach, gleich oder verschieden durch Fluor, Chlor, Brom, Methyl oder Trifluormethyl substituiertes Cyclopropyl, Cyclopentyl oder Cyclohexyl, für jeweils gegebenenfalls ein- bis dreifach, gleich oder verschieden durch Methyl, Methoxy, Chlor oder Trifluormethyl substituiertes Phenyl, Phenoxy, Phenylthio oder Phenylamino steht,

$R^7$ für Methyl, Ethyl, n- oder i-Propyl, n-, i-, s- oder t-Butyl, Chlormethyl, Dichlormethyl, Trichlormethyl, Trifluormethyl oder für gegebenenfalls ein- bis dreifach, gleich oder verschieden durch Methyl, Methoxy, Chlor oder Trifluormethyl substituiertes Phenyl steht,

X für Sauerstoff oder Schwefel steht,

n für eine Zahl 0, 1 oder 2 steht und

Py für jeweils ein- bis vierfach, gleich oder verschieden substituiertes 2-Pyridyl oder 4-Pyridyl steht, wobei als Substituenten in Frage kommen: Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl,

Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^9$,

wobei

$R^9$ für Amino, Methylamino, Ethylamino, Dimethylamino, Diethylamino, Fluordichlormethyl, Difluormethyl, Tetrafluorethyl, Trifluorchlorethyl, Trifluormethyl, Methyl oder Ethyl steht und

m für eine Zahl 0,1 oder 2 steht.

4. Verfahren zur Herstellung von 5-Amino-1-pyridyl-pyrazolen der Formel (I),

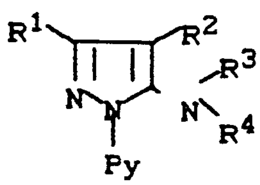

(I)

in welcher

$R^1$ für Wasserstoff oder für Alkyl mit 1 bis 12 Kohlenstoffatomen steht,

$R^2$ für Wasserstoff, Nitro, Nitroso, Halogen oder für den Rest $-\underset{\underset{O}{\|}}{C}-R^5$ steht, wobei

$R^5$ für Wasserstoff, Hydroxy, Alkyl, Alkenyl, Alkinyl, Halogenalkyl, Alkoxyalkyl, Alkylthioalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht,

$R^3$ für Wasserstoff, für einen Rest $-\overset{\overset{X}{\|}}{C}-R^6$ oder für einen Rest $-S(O)_n-R^7$ steht,

$R^4$ für Wasserstoff, für Alkyl, für einen Rest $-\overset{\overset{X}{\|}}{C}-R^6$ oder für einen Rest $-S(O)_n-R^7$ steht und für den Fall, daß $R^3$ für einen $-SO_2-R^7$-Rest steht auch für ein salzartig gebundenes anorganisches oder organisches Kation steht,

$R^6$ für Wasserstoff, Alkyl, Alkenyl, Alkinyl, Halogenalkyl, Alkoxyalkyl, Alkylthioalkyl, für gegebenenfalls substituiertes Cycloalkyl, für gegebenenfalls substituiertes Aryl, für Alkoxy, Alkylthio, für gegebenenfalls substituiertes Aryloxy, fürgegebenenfalls substituiertes Arylthio, für Alkylamino, Dialkylamino oder für gegebenenfalls substituiertes Arylamino steht,

X für Sauerstoff oder Schwefel steht,

n für eine Zahl 0, 1 oder 2 steht,

$R^7$ für Alkyl, Halogenalkyl oder für gegebenenfalls substituiertes Aryl steht und

Py für substituiertes C-verknüpftes Pyridyl steht,

dadurch gekennzeichnet, daß man

a) die erfindungsgemäßen 5-Amino-1-pyridyl-pyrazol-Derivate der Formel (Ia),

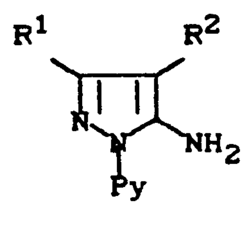

(Ia)

in welcher

$R^1$, $R^2$ und Py die oben angegebene Bedeutung haben,

erhält, wenn man Pyridylhydrazine der Formel (II),

$$Py - NH - NH_2 \qquad (II),$$

in welcher

Py die oben angegebene Bedeutung hat,

mit Acrylnitril-Derivaten der Formel (III),

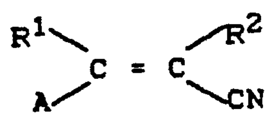

(III)

in welcher

$R^1$ und $R^2$ die oben angegebene Bedeutung haben und

A für Halogen, Hydroxy, Alkoxy oder Dialkylamino steht,

zunächst in einer 1. Stufe gegebenenfalls in Gegenwart eines Verdünnungsmittels und gegebenenfallsin Gegenwart eines Reaktionshilfsmittels umsetzt zu den Pyridylhydrazin-Derivaten der Formel (IV),

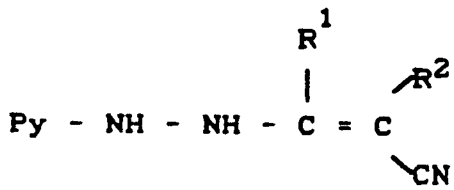

(IV)

in welcher

$R^1$, $R^2$ und Py die oben angegebene Bedeutung haben,

und diese in einer 2.Stufe, gegebenenfalls in Gegenwart eines Verdünnungsmittels und gegebenenfalls in Gegenwart eines Säurebindemittels cyclisiert,

oder daß man

b) die erfindungsgemäßen 5-Amino-1-pyridyl-pyrazol- Derivate der Formel (Ib),

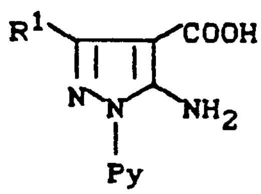

(Ib)

in welcher

$R^1$ und Py die oben angegebene Bedeutung haben,

erhält, wenn man 4-Alkoxycarbonyl-5-amino--pyrazole der Formel (Ir)

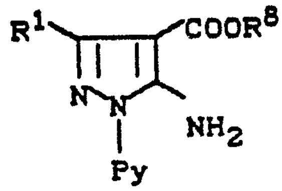

(Ir)

in welcher

$R^1$, $R^8$ und Py die oben angegebene Bedeutung haben,

an der Estergruppe in 4-Position des Pyrazolringes gegebenenfalls in Gegenwart eines Verdünnungsmittels und gegebenenenfalls in Gegenwart eines Katalysators, verseift, oder daß man

c) die erfindungsgemäßen 5-Amino-1-pyridyl-pyrazol- Derivate der Formel (Ic),

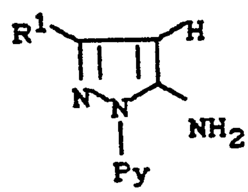

(Ic)

in welcher

$R^1$ und Py die oben angegebene Bedeutung haben,

erhält, wenn man 5-Amino-1-pyridyl-pyrazol-Derivate der Formel (Ib),

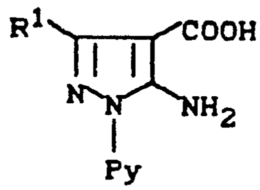

(Ib)

in welcher

$R^1$ und Py die oben angegebene Bedeutung haben,

gegebenenfalls in Gegenwart eines Verdünnungsmittels und gegebenenfalls in Gegenwart eines Katalysators, decarboxyliert,

oder daß man

d) die erfindungsgemäßen 5-Amino-1-pyridyl-pyrazol- Derivate der Formel (Id),

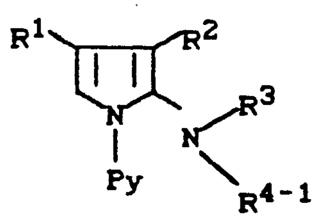

(Id)

in welcher

$R^{4-1}$ für Alkyl, für einen Rest $-\overset{X}{\overset{\|}{C}}-R^6$ oder für einen Rest $-S(O)_n-R^7$ steht und

$R^1$, $R^2$, $R^3$, $R^6$, $R^7$, X und n die oben angegebene Bedeutung haben,

erhält, wenn man 5-Amino-1-pyridyl-pyrazole der Formel (Is)

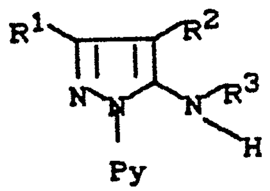

(Is)

in welcher

$R^1$, $R^2$, $R^3$ und Py die oben angegebene Bedeutung haben,

(d-α) mit Verbindungen der Formel (V),

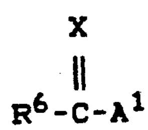

(V)

in welcher

$A^1$ für Halogen oder für einen Rest

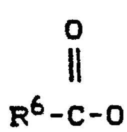

steht und

$R^6$ und X die oben angegebene Bedeutung haben, oder

(d-β) mit Verbindungen der Formel (Va),

$$R^7\text{-}S(O)_n\text{-}A^2 \qquad (Va)$$

in welcher

$A^2$ für Halogen steht und

$R^7$ und n die oben angegebene Bedeutung haben,

oder

(d-γ) mit Verbindungen der Formel (Vb),

$$R^8\text{-}A^3 \qquad (Vb)$$

in welcher

$R^8$ für Alkyl steht und

$A^3$ für Halogen, p-Toluolsulfonyloxy oder Alkoxysulfonyloxy steht,

gegebenenfalls in Gegenwart eines Verdünnungsmittels und gegebenenfalls in Gegenwart eines Säurebindemittels umsetzt,

oder daß man

e) die erfindungsgemäßen 5-Amino-1-pyridyl-pyrazol-Derivate der Formel (Ie),

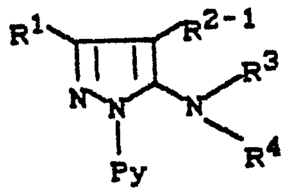

(Ie)

in welcher

$R^{2-1}$ für Halogen, Nitro, Nitroso, Formyl, Alkanoyl oder Aroyl steht und

$R^1$, $R^3$, $R^4$ und Py die oben angegebene Bedeutung haben,

erhält, wenn man 5-Amino-1-pyridyl-pyrazol-Derivate der Formel (It),

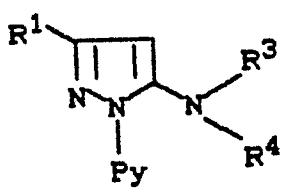

(It)

in welcher

$R^1$, $R^3$, $R^4$ und Py die oben angegebene Bedeutung haben,

mit elektrophilen Agenzien der Formel (VI),

$$R^{2-1}-A^4 \qquad (VI)$$

in welcher

$A^4$ für eine elektronenanziehende Abgangsgruppe steht und

$R^{2-1}$ die oben angegebene Bedeutung hat,

gegebenenfalls in Gegenwart eines Verdünnungsmittels und gegebenenfalls in Gegenwart eines Katalysators oder Reaktionshilfsmittels in 4-Stellung substituiert,

oder daß man

f) die erfindungsgemäßen 5-Amino-1-pyridyl-pyrazol- Derivate der Formel (If)

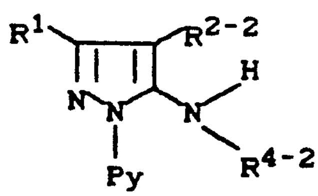

(If)

in welcher

$R^{2-2}$ für Halogen, Nitro oder Nitroso steht,

$R^{4-2}$ für Wasserstoff oder Alkyl steht und

$R^1$ und Py die oben angegebene Bedeutung haben,

erhält, wenn man 5-Acylamino-1-pyridyl-pyrazole der Formel (Iu)

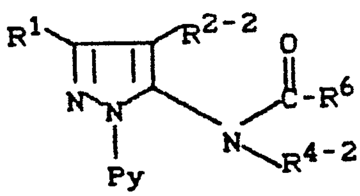

(Iu)

in welcher

$R^1$, $R^{2-2}$, $R^{4-2}$, $R^6$ und Py die oben angegebene Bedeutung haben,

gegebenenfalls in Gegenwart eines Verdünnungsmittels und gegebenenfalls in Gegenwart eines Katalysators an der Aminogruppe in 5-Position des Pyrazolringes deacyliert,

oder daß man

g) die erfindungsgemäßen 5-Amino-1-pyridyl-pyrazol-Derivate der Formel (Ig)

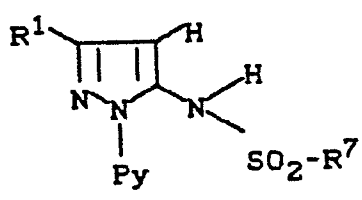

(Ig)

in welcher

$R^1$, $R^7$ und Py die oben angegebene Bedeutung haben,

erhält, wenn man 5-Bis-sulfonyl-amino-pyrazole der Formel (Iv)

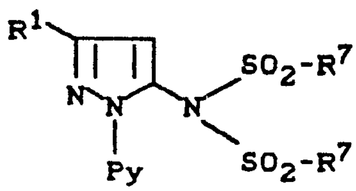

(Iv)

in welcher

$R^1$, $R^7$ und Py die oben angegebene Bedeutung haben,

mit Basen gegebenenfalls in Gegenwart eines Verdünnungsmittels spaltet,

oder daß man

h) die erfindungsgemäßen 5-Amino-1-pyridyl-pyrazol-Derivate der Formel (Ih)

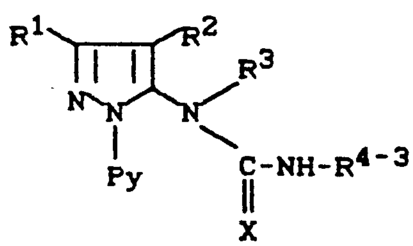

(Ih)

in welcher

$R^{4-3}$ für Alkyl oder für gegebenenfalls substituiertes Aryl steht und

$R^1$, $R^2$, $R^3$, X und Py die oben angegebene Bedeutung haben,

erhält, wenn man 5-Amino-1-pyridyl-pyrazole der Formel (Is),

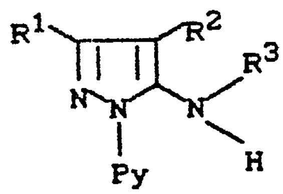

(Is)

in welcher

$R^1$, $R^2$, $R^3$ und Py die oben angegebene Bedeutung haben,

mit Iso(thio)cyanaten der Formel (VII),

$$R^{4-3} - N = C = X \qquad (VII)$$

in welcher

$R^{4-3}$ und X die oben angegebene Bedeutung haben,

gegebenenfalls in Gegenwart eines Verdünnungsmittels und gegebenenfalls in Gegenwart eines Säurebindemittels umsetzt,

oder daß man

i) die erfindungsgemäßen 5-Amino-1-pyridyl-pyrazol-Derivate der Formel (Ii),

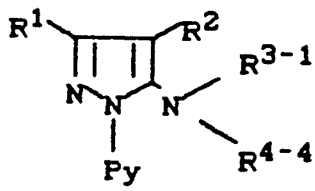

(Ii)

in welcher

$R^{4-4}$ für Alkyl steht,

$R^{3-1}$ für Wasserstoff oder Alkyl steht und

$R^1$, $R^2$ und Py die oben angegebene Bedeutung haben,

erhält, wenn man 5-Halogen-pyrazole der Formel (VIII),

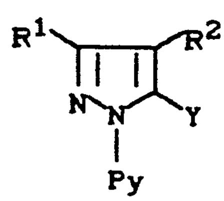

(VIII)

in welcher

Y für Halogen steht und

$R^1$, $R^2$ und Py die oben angegebene Bedeutung haben,

mit Aminen der Formel (IX),

$R^{4-4}$-NH
|
$R^{3-1}$ (IX)

in welcher

$R^{4-4}$ für Alkyl steht und

$R^{3-1}$ für Wasserstoff oder Alkyl steht,

gegebenenfalls in Gegenwart eines Verdünnungsmittels und gegebenenfalls in Gegenwart eines Säurebindemittels umsetzt,

oder daß man

k) die erfindungsgemäßen 5-Amino-1-pyridyl-pyrazol-Derivate der Formel (Ik),

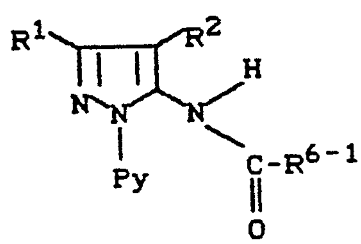

(Ik)

in welcher

$R^{6-1}$ für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht, und

$R^1$, $R^2$ und Py die oben angegebene Bedeutung haben,

erhält, wenn man (Bis)Carbamate der Formel (Iw),

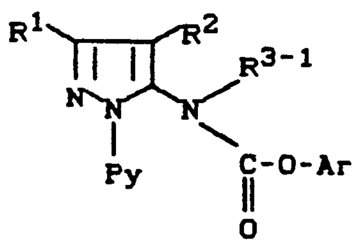

(Iw)

in welcher

$R^{3-1}$ für Wasserstoff oder für einen Rest

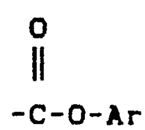

steht, wobei

Ar für gegebenenfalls substituiertes Aryl steht und

$R^1$, $R^2$ und Py die oben angegebene Bedeutung haben,

mit Verbindungen der Formel (X),

$$R^{6-1} - H \qquad (X)$$

in welcher

$R^{6-1}$ die oben angegebene Bedeutung hat,

gegebenenfalls in Gegenwart eines Verdünnungsmittels und gegebenenfalls in Gegenwart eines basischen Katalysators umsetzt,
oder daß man

1) Salze von erfindungsgemäßen 5-Sulfonamido-pyrazol-Derivaten der Formel (Ix) erhält,

wenn man 5-Sulfonamido-pyrazole der Formel (Ix),

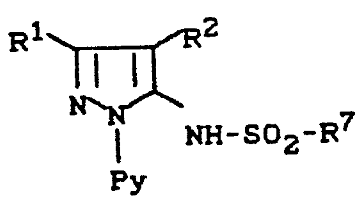

(Ix)

in welcher

$R^1$, $R^2$, $R^7$ und Py die oben angegebene Bedeutung haben,

0207285

entweder mit Salzen der Formel (XI),

$$M^{\oplus} - G^{\ominus} \qquad (XI)$$

in welcher

$M^{\oplus}$ für ein Äquivalent eines anorganischen oder organischen Kations steht und

$G^{\ominus}$ für ein Äquivalent eines geeigneten Gegenions steht,

oder mit primären, sekundären oder tertiären Aminen gegebenenfalls in Gegenwart eines Verdünnungsmittel umsetzt.

5. Herbizide Mittel, gekennzeichnet durch einen Gehalt an mindestens einem 5-Amino-1-pyridyl-pyrazol der Formel (I) gemäß den Ansprüchen 1 bis 4.

6. Verfahren zur Bekämpfung von Unkräutern, dadurch gekennzeichnet, daß man ein 5-Amino-1-pyridyl-pyrazol der Formel (I) gemäß den Ansprüchen 1 bis 4, auf Unkräuter und/oder ihren Lebensraum einwirken läßt.

7. Verwendung von 5-Amino-1-pyridyl-pyrazolen der Formel (I) gemäß den Ansprüchen 1 bis 4, zur Bekämpfung von Unkräutern.

8. Verfahren zur Herstellung von herbiziden Mitteln, dadurch gekennzeichnet, daß man 5-Amino-1-pyridyl-pyrazole der Formel (I) gemäß den Ansprüchen 1 bis 4 mit Streckmitteln und/oder oberflächenaktiven Mitteln vermischt.

9. 5-Halogenpyrazole der Formel (VIIIa),

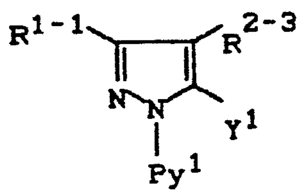

(VIIIa)

in welcher

$R^{1-1}$ für Wasserstoff oder für Alkyl mit 1 bis 12 Kohlenstoffatomen steht,

$R^{2-3}$ für Wasserstoff, Nitro, Nitroso, Halogen oder für einen Rest $-\underset{\underset{O}{\|}}{C}-R^5$ steht, wobei

$R^5$ für Wasserstoff, Hydroxy, Alkyl, Alkenyl, Alkinyl, Halogenalkyl, Alkoxyalkyl, Alkylthioalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht,

$Y^1$ für Halogen steht und

$Py^1$ für substituiertes C-verknüpftes Pyridyl steht,

wobei jedoch für den Fall, daß gleichzeitig $R^{1-1}$ für Methyl, $R^{2-3}$ für Wasserstoff und $Y^1$ für Chlor steht, $Py^1$ nicht für den 5-Nitro-2-pyridyl-Rest steht.

10. Verfahren zur Herstellung von 5-Halogenpyrazolen der Formel (VIIIa),

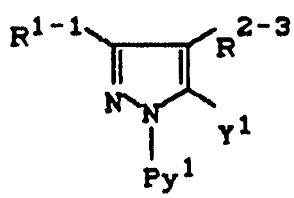

(VIIIa)

in welcher

$R^{1-1}$ für Wasserstoff oder für Alkyl mit 1 bis 12 Kohlenstoffatomen steht,

$R^{2-3}$ für Wasserstoff, Nitro, Nitroso, Halogen oder für einen Rest $-\underset{\underset{O}{\|}}{C}-R^5$ steht, wobei

$R^5$ für Wasserstoff, Hydroxy, Alkyl, Alkenyl, Alkinyl, Halogenalkyl, Alkoxyalkyl, Alkylthioalkyl, für gegebenenfalls substituiertes Cycloalkyl, für gegebenenfalls substituiertes Aryl, für Alkoxy, Alkylthio, für gegebenenfalls substituiertes Aryloxy, für gegebenenfalls substituiertes Arylthio, für Alkylamino, Dialkylamino oder für gegebenenfalls substituiertes Arylamino steht,

$Y^1$ für Halogen steht und

$Py^1$ für substituiertes C-verknüpftes Pyridyl steht,

wobei jedoch für den Fall, daß gleichzeitig $R^{1-1}$ für Methyl, $R^{2-3}$ für Wasserstoff und $Y^1$ für Chlor steht, $Py^1$ nicht für den 5-Nitro-2-pyridyl-Rest steht, dadurch gekennzeichnet, daß man

a) Alkoxymethylen-malonester der Formel (XIV),

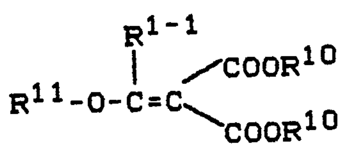

(XIV)

in welcher

$R^{1-1}$ die oben angegebene Bedeutung hat und

$R^{10}$ und $R^{11}$ unabhängig voneinander jeweils für Alkyl, insbesondere für Methyl oder Ethyl stehen,

mit Pyridylhydrazinen der Formel (IIa)

$$Py^1 - NH - NH_2$$

in welcher

$Py^1$ die oben angegebene Bedeutung hat,

zunächst in einer ersten Stufe gegebenenfalls in Gegenwart eines Verdünnungsmittels bei Temperaturen zwischen +10° C und +80° C umsetzt, und die so erhältlichen Pyrazolcarbonsäureester der Formel (XV)

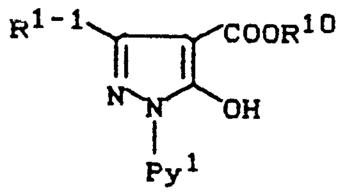

(XV)

in welcher

$R^{1-1}$, $R^{10}$ und $Py^1$ die oben angegebene Bedeutung haben,

in einer 2. Stufe gegebenenfalls in Gegenwart eines Verdünnungsmittels und gegebenenfalls in Gegenwart einer Base bei Temperaturen zwischen +30° C und +70° C decarboxyliert zu Pyrazolinonen der Formel (XVI),

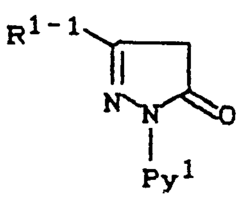

(XVI)

in welcher

$R^{1-1}$ und $Py^1$ die oben angegebene Bedeutung haben,

und diese in einer 3. Stufe mit Halogenierungsmitteln, umsetzt, und gegebenenfalls in einer 4. Stufe die so erhältlichen 5-Halogen-pyrazole der Formel (VIIIb),

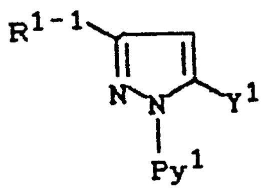

(VIIIb)

in welcher

$R^{1-1}$, $Y^1$ und $Py^1$ die oben angegebene Bedeutung haben,

mit elektrophilen Agenzien der Formel (VI)

$$R^{2-1}-A^4 \quad (VI)$$

in welcher

$R^{2-1}$ für Halogen, Nitroso, Nitro, Formyl, Alkanoyl oder Aroyl steht und

$A^4$ für eine elektronenanziehende Abgangsgruppe steht,

gegebenenfalls in Gegenwart eines Verdünnungsmittels, und gegebenenfalls in Gegenwart eines Katalysators oder Reaktionshilfsmittels, in 4-Stellung substituiert,

oder daß man

b) 5-Amino-1-pyridyl-pyrazole der Formel (Ia-1)

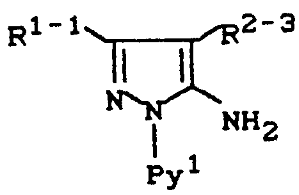

(Ia-1)

in welcher

$R^{1-1}$, $R^{2-3}$ und $Py^1$ die oben angegebenen Bedeutungen haben,

in Gegenwart eines Diazotierungsmittels mit Haloform-Verbindungen umsetzt.

0207285

Europäisches Patentamt

# EUROPÄISCHER RECHERCHENBERICHT

Nummer der Anmeldung

EP 86 10 7125

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
| Y | GB-A-2 136 427 (MAY & BAKER LTD.) * Ansprüche 1, 7, 11, 21 * --- | 1,4-8 | C 07 D 401/04<br>A 01 N 43/56 |
| Y,D | EP-A-0 034 945 (MAY & BAKER LTD.) * Seite 3, Zeile 12 - Seite 4, Zeile 24, besonders Seite 4, Zeilen 10-12; Seiten 43-51 * --- | 1,4-8 | |
| A,D | DE-A-3 129 429 (BASF A.G.) * Ansprüche * --- | 1,4-8 | |
| A | GB-A- 893 755 (CIBA LTD.) * Beispiele 3, 4 * --- | 1,4 | |
| A | CHEMICAL ABSTRACTS, Band 69, Nr. 15, 7. Oktober 1968, Seite 5530, Spalte 1, Abstract Nr. 59155g, Columbus, Ohio, US; I.I. GRANDBERG et al.: "Pyrazoles. LXII. The synthesis of 5-aminopyrazole series with potential physiological activity", & KHIM.-FARM.ZH. 1968, (1), 16-22 --- -/- | 1 | RECHERCHIERTE SACHGEBIETE (Int Cl 4)<br>C 07 D 401/00<br>A 01 N 43/00 |

Der vorliegende Recherchenbericht wurde für alle Patentansprüche erstellt.

| Recherchenort | Abschlußdatum der Recherche | Prüfer |
|---|---|---|
| BERLIN | 08-09-1986 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

EP 86 10 7125

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.4) |
|---|---|---|---|
| A | CHEMICAL ABSTRACTS, Band 94, Nr. 23, 8. Juni 1981, Seite 650, Spalte 2, Abstract Nr. 192215j, Columbus, Ohio, US; A.M. KHAN et al.: "Hetarylpyrazoles. II. Reactions of pyrazol-1'-ylpyridines", & J. HETEROCYCL. CHEM. 1981, 9-14, in Zusammenhang mit FORMULA INDEX, Band 94, Januar-Juni 1981, Seite 535F, Spalte 2, Zeilen 74, 75 : Pyridine, 2-(5-chloro-3-methyl-1h-pyrazol-1-gl)-5-nitro" --- | 9 | |
| P,X | EP-A-0 151 867 (ELI LILLY AND CO.) * Seite 3, Zeile 19 - Seite 9, Zeile 2; Beispiel 17 in Zusammenhang mit Beispiele 1A, B und Seite 23, Zeilen 15, 16 * ----- | 1-5,9-10 | |
| | | | RECHERCHIERTE SACHGEBIETE (Int Cl.4) |

Der vorliegende Recherchenbericht wurde für alle Patentansprüche erstellt

| Recherchenort | Abschlußdatum der Recherche | Prüfer |
|---|---|---|
| BERLIN | 08-09-1986 | VAN AMSTERDAM L.J.P. |

KATEGORIE DER GENANNTEN DOKUMENTE

X von besonderer Bedeutung allein betrachtet
Y von besonderer Bedeutung in Verbindung mit einer anderen Veröffentlichung derselben Kategorie
A technologischer Hintergrund
O nichtschriftliche Offenbarung
P Zwischenliteratur
T der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument